(19) Europäisches Patentamt
European Patent Office
Office européen des brevets

(11) Publication number: **0 316 769**
**A2**

(12) # EUROPEAN PATENT APPLICATION

(21) Application number: **88118754.6**

(51) Int. Cl.[4]: **C07K 7/00 , A61K 37/02**

(22) Date of filing: **10.11.88**

Claim for the following Contracting State: ES.

(30) Priority: **13.11.87 US 120056**

(43) Date of publication of application: **24.05.89 Bulletin 89/21**

(84) Designated Contracting States: **AT BE CH DE ES FR GB GR IT LI LU NL SE**

(71) Applicant: **G.D. Searle & Co.**
**P.O. Box 5110**
**Chicago Illinois 60680(US)**

(72) Inventor: **Bovy, Philippe R.**
**13 Courtway Place**
**St. Louis Missouri 63109(US)**
Inventor: **O'Neal, Joan M.**
**5536 Rosa Avenue**
**St. Louis Missouri 63109(US)**
Inventor: **Olins, Gillian M.**
**16464 Birch Forest Drive**
**Ballwin Missouri 63011(US)**

(74) Representative: **Beil, Hans Christoph, Dr. et al**
**Beil, Wolff und Beil Rechtsanwälte**
**Adelonstrasse 58**
**D-6230 Frankfurt am Main 80(DE)**

(54) **Disulfide bridged cyclic peptides containing a cgridri sequence useful in control of hypertension.**

(57) Disulfide bridged cyclic peptides are described which are preferably 13 to 20 amino acid residues in length and which include the endocyclic sequence cys-gly-arg-ile-asp-arg-ile. Peptides of this class are selective for and show picomolar-range affinity for the non-guanyl cyclase-coupled atrial peptide receptor. Affinity for this receptor is associated with potentiation of the mean arterial pressure response to atrial peptide and these peptides are therefore useful in control of hypertension. Peptides of most interest are of the formula

$$\text{A-}X^1_m\text{-cys-gly-arg-ile-asp-arg-ile-}X^2_n\text{-gly-cys-}X^3_r\text{-B}$$

wherein $X^1$ is the peptidic fragment ser-ser; wherein $X^2$ is a peptidic fragment selected from gly-ser-gly-leu, gly-ala-gly-leu and gly-leu; wherein $X^3$ is the peptidic fragment asn-ser-phe-arg; wherein m is zero or one, n is one and r is one; and wherein A represents an amino terminus or its pharmaceutically-acceptable salt, and B represents a carboxyl terminus or its pharmaceutically-acceptable ester, amide or salt.

EP 0 316 769 A2

# DISULFIDE BRIDGED CYCLIC PEPTIDES CONTAINING A CGRIDRI SEQUENCE USEFUL IN CONTROL OF HYPERTENSION

## FIELD OF THE INVENTION

This invention is in the field of cardiovascular therapeutics and relates to peptides useful for control of hypertension. Of particular interest are disulfide bridged cyclic peptides which potentiate mean arterial pressure response to naturally-occurring or synthetic atrial peptides.

## BACKGROUND OF THE INVENTION

Crude extracts of rat atria containing potent diuretic and natriuretic substances have been previously referred to as atrial natriuretic factor [A.J. deBold et al, Life Sci., 28, 89-94 (1981)]. These substances have subsequently been chemically defined as peptides, commonly referred to as atrial peptides or AP.

Various peptides of related structures have been isolated, sequenced and shown to have natriuretic, diuretic and vasorelaxant activity in varying degrees. A group of atrial peptides of significant interest, known as Atriopeptins I, II and III (AP-I, AP-II and AP-III), are described, for example, in a publication of Currie et al, Science 223, 67-69 (1984), in a publication of Geller et al, Biochem. Biophys. Res. Commun., 120 (2), 333-338 (1984), and in U.S. Patent 4,496,554 to Needleman. These peptides are in the oxidized, that is, cyclized form, and have the following amino acid sequences:

**ATRIOPEPTIN I:**

Ser-ser-cys-phe-gly-gly-arg-ile-asp-arg-ile-gly-ala-gln-ser-

gly-leu-gly-cys-asn-ser;

**ATRIPEPTIN II:**

Ser-ser-cys-phe-gly-gly-arg-ile-asp-arg-ile-gly-ala-gln-ser-

gly-leu-gly-cys-asn-ser-phe-arg.

**ATRIOPEPTIN III:**

Ser-ser-cys-phe-gly-gly-arg-ile-asp-arg-ile-gly-ala-gln-ser-

gly-leu-gly-cys-asn-ser-phe-arg-tyr.

Various modifications of AP-III have also been described. Thus, the 28 amino acid peptide ser-leu-arg-arg-AP-III (SLRR-AP-III), also known as Cardionatrin I, is disclosed in European Patent Application 116,784, published August 29, 1984. The human analogue of the 28 amino acid Cardionatrin I having a $met^{12}$ replacement for $ile^{12}$ is described by Kangawa and Matsuo, Biochem. Biophys. Res. Commun., 118 (1), 131-139 (1984).

The 26 amino acid peptide arg-arg-AP-III, also known as atrial natriuretic factor or ANF (8-33), is disclosed as a fragment of a larger 33 amino acid peptide by Seidah et al, Proc. Nat'l. Acad. Sci. USA, 81, 2640-44 (1984).

The 25 amino acid analog of AP-III having an arg extension at the amino terminus, also known as auriculin, is described by Yamanaka et al., Nature, 309, 719-22 (1984).

The common designation "AP" has been used for referring to any peptide of the family of atrial peptides or atriopeptins.All of these peptides have a common amino acid sequence but may differ in length.

Atrial peptides have therapeutic potential in the treatment of congestive heart failure and hypertension and have been recognized as important for regulation of body fluid volume and blood pressure. Some of the physiological effects of atrial peptides are well-known. For example, AP-III has a natriuretic and diuretic effect and it inhibits the contractile response of vascular smooth muscle to several agents. Also, AP-III blocks angiotensin II-stimulated aldosterone secretion from the adrenal cortex and inhibits renin release. AP-III acts through specific receptors at its target tissues. AP-III induces activation of particulate guanylate cyclase and increases cyclic GMP formation [J. Tremblay et al, FEBS Lett., 181, 17-22 (1985); S.A. Waldman et al, J. Biol. Chem., 259, 14332-14334 (1984)].

The variety of functions for atrial peptides suggest the existence of multiple, functionally-distinct AP receptors [D.B. Shenck et al, J. Biol. Chem., 260, 14887-14890 (1985)]. In fact, several studies using affinity labeling techniques [D.C. Leitman et al, J. Biol. Chem., 261, 11650-11655 (1986)] suggest the existence of multiple AP receptor sub-types. However, the relationship between multiple AP receptors and distinct physiological functions is not evident thus far.

At least two different types of AP receptors have been identified recently [R. Takayanagi et al, J. Biol. Chem., 262, 12104-12113 (1987)]. One of the receptors is coupled to the particulate guanyl cyclase ("guanyl cyclase-coupled receptor") while the second is uncoupled to the cyclase ("guanyl cyclase-free receptor").The relative populations of the two receptors vary with the tissues and the species. AP-III binds with equal affinity to both receptors. AP-I binds with some selectivity to the guanyl cyclase-free receptor as shown by affinity cross-linking techniques and binding experiments [D. C. Leitman et al, J. Biol. Chem., 261, 11650-55 1986)], but has a 1.1- to about 5-fold reduced affinity with respect to AP-III [R.M. Scarborough et al, J. Biol. Chem., 261, 12960-64 (1986)]. In a later series of publications [Suzuki et al, 2nd Annual Meeting of the American Society of Hypertension, Abstract B60, 187 (1987)], several analogues of AP were reported to bind to the bovine aortic smooth muscle AP receptors, but fail to elicit increase of cGMP; also observed was slight potentiation of the hemodynamic and renal excretory effects of SLRR-AP-III, when infused at concentrations of 5 to 10 µg/min. Other reports on divergence of AP analog cGMP response in smooth muscle cells and adrenal zona glomerulosa cells were published as evidence for receptor sub-types [G.P. Budzik et al, Biochem. Biophys. Res. Commun., 144, 422-31 (1987); R. Takayanagi et al, Biochem. Biophys. Res. Commun., 144, 244-250 (1987)].

EP Application No. 223,143, published May 27, 1987, describes a large class of several hundred peptides or modified peptides, a predominant number of which are characterized by inclusion of the pentapeptide sequence arg-ile-asp-arg-ile. These peptides are mentioned as having high affinity for bovine aortic smooth muscle receptor sites which may be involved in the clearance and removal of atrial peptides.It is further stated that the effect of these peptides *in vivo* is due to their ability to potentiate the effect of endogenous ANP, possibly through blockade of the receptors involved in the binding and clearance of endogenous atrial peptide.

Salk Institute PCT Application US85/00746 describes a class of peptides of 18 to 24 amino acid residues characterized by inclusion of the pentapeptide sequence arg-ile-asp-arg-ile connected to the oligopeptide ala-glu-ser by either a glycine or D-alanine residue. These peptides are characterized as atrial peptide analogues that are more potent and longer lasting than AP-I or AP-II in functioning as a diuretic or a smooth muscle relaxant.

## DESCRIPTION OF THE INVENTION

Treatment of hypertension is accomplished by administering to a mammal susceptible to hypertension a therapeutically-effective amount of a compound of a class of disulfide bridged cyclic peptides represented by Formula I:

$$A\text{-}X^1_m\text{-}\underline{cys\text{-}gly\text{-}arg\text{-}ile\text{-}asp\text{-}arg\text{-}ile\text{-}X^2_n\text{-}gly\text{-}cys}\text{-}X^3_r\text{-}B \qquad (I)$$

wherein

$X^1$ is a fragment consisting of one to about eight amino acid residues selected from arg, ser, pro and leu, and wherein m is an integer selected from zero through four;

$X^2$ is a fragment consisting of one to about six amino acid residues selected from gly, leu, ser, ala and gln,

and wherein n is an integer selected from zero through three;
$X^3$ is a fragment consisting of one to about six amino acid residues selected from lys, asn, ser, phe, arg, tyr and $^{125}$I-tyr, wherein r is an integer selected from zero through four;
wherein A represents an amino terminus or its pharmaceutically acceptable salt and wherein B represents a carboxyl terminus or its pharmaceutically-acceptable ester, amide or salt;
with the proviso that the sum of all amino acid residues in said peptide is an integer in a range from 13 through 25.

A first sub-class of preferred compounds of Formula I consists of those wherein $X^1$ is a peptidic fragment consisting of one to eight amino acid residues and m is zero or one. More preferred are those compounds wherein $X^1$ is a fragment consisting of one to six amino acid residues selected from ser, arg and leu. Even more preferred are those compounds wherein $X^1$ is a fragment consisting of one to four amino acid residues selected from ser and arg. Most preferred within this first sub-class of Formula I are compounds wherein $X^1$ is a fragment consisting of one or two serine residues.

A second sub-class of preferred compounds consists of those compounds wherein $X^2$ is a peptidic fragment consisting of two to six amino acid residues and n is one. More preferred are those compounds wherein $X^2$ is a fragment consisting of two or four amino-acid residues selected from gly-ala-gly-leu, gly-ser-gly-leu, gly-ala-gln-leu, gln-ser-gly-leu, gly-ala-gln-ser, gln-leu, gly-ser and gly-ala. Where $X^2$ is a fragment consisting of four amino-acid residues, then most preferred is a fragment selected from gly-ser-gly-leu and gly-ala-gly-leu. Where $X^2$ is a fragment consisting of two amino-acid residues, then most preferred is the fragment gly-leu. Also, the most preferred peptides are characterized by the absence of glutamine in the $X^2$ fragment.

A third sub-class of preferred compounds of Formula I consists of those compounds wherein $X^3$ is a peptidic fragment consisting of one to six amino-acid residues and r is zero or one. More preferred are those compounds wherein $X^3$ is a fragment consisting of four or five amino-acid residues. Where $X^3$ is a five amino acid fragment, then most preferred is the fragment asn-ser-phe-arg-tyr.Where $X^3$ is a fragment consisting of four amino-acid residues, then most preferred are residues selected from asn, ser, phe and arg. A particularly preferred four amino acid fragment is asn-ser-phe-arg. Even more preferred are peptides having a C-terminus arginineamide.

A fourth sub-class of preferred compounds of Formula I consists of those compounds wherein $X^1$ is selected from arg-arg, arg-ser and ser-ser; wherein $X^2$ is selected from gly-ser-gly-leu, gly-ala-gly-leu, gln-ser-gly-leu, gly-ala-gln-ser, gly-ala-gln-leu, gly-ser, gln-leu, gly-ala and gly-leu; wherein $X^3$ is selected from asn-ser-phe, asn-ser-phe-arg, lys-ser-phe-arg, asn-ser-phe-arg-tyr, lys-ser-phe-arg-tyr, asn-ser-phe-arg-$^{125}$I-tyr, and lys-ser-phe-arg-$^{125}$I-tyr; wherein m is zero or one, n is one, and r is zero or one.A more preferred group of compounds within the fourth sub-class of Formula I consists of those compounds wherein $X^1$ is selected from arg-arg, arg-ser and ser-ser; wherein $X^2$ is selected from gly-ser-gly-leu, gly-ser, gln-leu and gly-ala; wherein $X^3$ is selected from asn-ser-phe-arg, lys-ser-phe-arg, asn-ser-phe-arg-tyr and lys-ser-phe-arg-tyr; wherein m is zero or one, n is one, and r is zero or one. Even more preferred are those compounds wherein $X^1$ is selected from arg-arg, arg-ser and ser-ser; wherein $X^2$ is gly-ser-gly-leu; and wherein $X^3$ is selected from asn-ser-phe-arg-tyr and lys-ser-phe-arg-tyr. A particularly preferred compound is the peptide

**cys-gly-arg-ile-asp-arg-ile-gly-ser-gly-leu-gly-cys-$NH_2$.**

Another particularly preferred compound is the peptide

**ser-ser-cys-gly-arg-ile-asp-arg-ile-gly-ser-gly-leu-gly-cys-asn-**

**ser-phe-arg-tyr.**

A fifth sub-class of preferred compounds of Formula I consists of those peptides wherein $X^1$ is selected from arg-arg, arg-ser and ser-ser; wherein $X^2$ is selected from gly-ser, gly-leu, gln-leu and gly-ala; wherein $X^3$ is selected from asn-ser-phe-arg, lys-ser-phe-arg, asn-ser-phe-arg-tyr, lys-ser-phe-arg-tyr, asn-ser-phe-arg-$^{125}$I-tyr and lys-ser-phe-arg$^{125}$tyr; wherein m is zero or one, n is one and r is one. Of this fifth sub-class when $X^3$ is selected from asn-ser-phe-arg and lys-ser-phe-arg, then it is more preferred that the B terminus is a primary amide or a lower alkyl secondary or tertiary amide moiety. "Lower alkyl" means linear or

branched radicals having one to about eight carbon atoms.

Another more preferred group of compounds within the fifth sub-class of Formula I consists of those peptides wherein $X^1$ is selected from arg-arg, arg-ser and ser-ser; wherein $X^2$ is gly-ser-gly-leu; and wherein $X^3$ is selected from asn-sr-phe-arg and lys-ser-phe-arg. A particularly preferred peptide is

**cys-gly-arg-ile-asp-arg-ile-gly-ser-gly-leu-gly-cys-asn-ser-phe-**
**arg-$NH_2$.**

A sixth sub-class of preferred compounds of Formula I consists of those compounds wherein $X^1$ is selected from arg-arg, arg-ser and ser-ser; wherein $X^2$ is selected from gly-ser-gly-leu, gly-ala-gly-leu, gln-ser-gly-leu, gly-ala-gln-ser, gly-ala-gln-leu, gly-leu, gly-ala, gly-ser and gln-leu; wherein $X^3$ is selected from asn-ser-phe-arg and lys-ser-phe-arg; and wherein each of m, n and r is one. A more preferred group of compounds within this sixth sub-class of Formula I consists of those peptides wherein $X^1$ is ser-ser; wherein $X^2$ is selected from gly-ser-gly-leu, gly-ala-gly-leu, gln-ser-gly-leu, gly-ala-gln-ser and gly-ala-gln-leu; and wherein $X^3$ is asn-ser-phe-arg. Particularly preferred are the following peptides:

**ser-ser-cys-gly-arg-ile-asp-arg-ile-gln-ser-gly-leu-gly-cys-asn-**
**ser-phe-arg-$NH_2$;**

**ser-ser-cys-gly-arg-ile-asp-arg-ile-gly-ala-gln-ser-gly-cys-asn-**
**ser-phe-arg-$NH_2$;**

**ser-ser-cys-gly-arg-ile-asp-arg-ile-gly-ala-gln-leu-gly-cys-asn-**
**ser-phe-arg-$NH_2$;**

**ser-ser-cys-gly-arg-ile-asp-arg-ile-gly-ser-gly-leu-gly-cys-asn-**
**ser-phe-arg-$NH_2$;**

**ser-ser-cys-gly-arg-ile-asp-arg-ile-gly-ala-gly-leu-gly-cys-asn-**
**ser-phe-arg-$NH_2$.**

A seventh sub-class of preferred compounds of Formula I consists of those compounds wherein $X^1$ is selected from arg-arg, arg-ser and ser-ser; wherein $X^2$ is selected from gln-leu, gly-ser, gly-ala and gly-leu; and wherein $X^3$ is selected from asn-ser-phe-arg and lys-ser-phe-arg; and wherein each of m, n and r is one. A more preferred group of compounds within this seventh sub-class of Formula I consists of those peptides wherein $X^1$ is ser-ser; and $X^2$ is selected from gln-leu, gly-ser, gln-ala and gly-leu; wherein $X^3$ is selected from asn-ser-phe-arg and lys-ser-phe-arg.
Particularly preferred are the following peptides:

ser-ser-cys-gly-arg-ile-asp-arg-ile-gln-leu-gly-cys-asn-ser-phe-arg-$NH_2$;

ser-ser-cys-gly-arg-ile-asp-arg-ile-gly-ser-gly-cys-asn-ser-phe-arg-$NH_2$;

ser-ser-cys-gly-arg-ile-asp-arg-ile-gly-ala-gly-cys-asn-ser-phe-arg-$NH_2$;

ser-ser-cys-gly-arg-ile-asp-arg-ile-gly-leu-gly-cys-asn-ser-phe-arg-$NH_2$.

A class of particularly preferred peptides of Formula I consists of those peptides wherein m is zero or one, n is one, and r is zero or one. Representative peptides of this class are listed in Table I.

TABLE I

| Peptide No. | Formula |
|---|---|
| 1 | ser-ser-cys-gly-arg-ile-asp-arg-ile-gly-ser-gly-leu-gly-cys-asn-ser-phe-arg-tyr |
| 2 | cys-gly-arg-ile-asp-arg-ile-gly-ser-gly-leu-gly-cys-$NH_2$ |
| 3 | ser-ser-cys-gly-arg-ile-asp-arg-ile-gln-ser-gly-leu-gly-cys-asn-ser-phe-arg-$NH_2$ |
| 4 | ser-ser-cys-gly-arg-ile-asp-arg-ile-gly-ala-gln-leu-gly-cys-asn-ser-phe-arg-$NH_2$ |
| 5 | ser-ser-cys-gly-arg-ile-asp-arg-ile-gly-ala-gln-ser-gly-cys-asn-ser-phe-arg-$NH_2$ |
| 6 | ser-ser-cys-gly-arg-ile-asp-arg-ile-gln-leu-gly-cys-asn-ser-phe-arg-$NH_2$ |
| 7 | ser-ser-cys-gly-arg-ile-asp-arg-ile-gly-ser-gly-cys-asn-ser-phe-arg-$NH_2$ |
| 8 | ser-ser-cys-gly-arg-ile-asp-arg-ile-gly-ala-gly-cys-asn-ser-phe-arg-$NH_2$ |
| 9 | ser-ser-cys-gly-arg-ile-asp-arg-ile-gly-ser-gly-leu-gly-cys-asn-ser-phe-arg-$NH_2$ |
| 10 | ser-ser-cys-gly-arg-ile-asp-arg-ile-gly-ala-gly-leu-gly-cys-asn-ser-phe-arg-$NH_2$ |

TABLE I

| Peptide No. | Formula |
|---|---|
| 11 | cys-gly-arg-ile-asp-arg-ile-gly-ser-gly-leu-gly-cys-asn-ser-phe-arg-$NH_2$ |
| 12 | ser-ser-cys-gly-arg-ile-asp-arg-ile-gly-leu-gly-cys-asn-ser-phe-arg-$NH_2$ |

Also included in the scope of the present invention are compounds which are intermediates to the peptides of Formula I. These intermediate compounds are represented by Formula II:

$$A\text{-}X^1_m\text{-}\underset{\textcircled{P'}}{\text{cys}}\text{-gly-arg-ile-asp-arg-ile-}X^2_n\text{-gly-}\underset{\textcircled{P'}}{\text{cys}}\text{-}X^3_r\text{-B} \quad \text{(II)}$$

wherein

$X^1$ is a peptidic fragment consisting of one to about eight amino acid residues selected from arg, ser, pro and leu, and wherein m is an integer selected from zero through four;

$X^2$ is a peptidic fragment consisting of one to about six amino acid residues selected from gly, leu, ser, ala and gln, and wherein n is an integer selected from zero through three;

$X^3$ is a peptidic fragment consisting of one to about six amino acid residues selected from lys, asn, ser, phe, arg and tyr, wherein r is an integer selected from zero through four;

wherein A represents an amino terminus or its pharmaceutically-acceptable salt and wherein B represents a carboxyl terminus or its pharmaceutically-acceptable ester, amide or salt; wherein (P') is an hydrido or a protecting group used as protection for the sulfhydryl function of the cysteine residue; and wherein the cysteine residues may or may not be simultaneously protected; with the proviso that the sum of all amino acid residues in said peptide is an integer in a range from about 13 through about 25. A preferred protecting group is acetamidomethyl group.

A preferred group of intermediate compounds within Formula II consists of those compounds wherein $X^1$ is selected from arg-arg, arg-ser and ser-ser; wherein $X^2$ is selected from gly-ser-gly-leu, gly-ala-gly-leu, gln-ser-gly-leu, gly-ala-gln-ser, gly-ala-gln-leu, gly-ser, gln-leu and gly-ala; wherein $X^3$ is selected from asn-ser-phe, lys-ser-phe, asn-ser-phe-arg, lys-ser-phe-arg, asn-ser-phe-arg-tyr and lys-ser-phe-arg-tyr; and wherein m is zero or one, n is one, and r is zero or one.

All compounds of Formulas I and II are characterized in having a sequence of amino-acid residues cys-gly-arg-ile-asp-arg-ile, called the "CGRIDRI sequence". The compounds of Formula I are also further characterized by the absence of an amino-acid residue having an aromatic side chain within the endocyclic moiety which includes the CGRIDRI sequence. The compounds of Formula II are characterized by the absence of aromatic amino-acid residues between the $X^1$ and $X^2$ substituents. All of these compounds are still further characterized in being about 13 to about 25 amino-acid residues in length. Preferred peptides consist of 13 to 20 amino-acid residues.

Peptides of Formulas I and II can be assembled from amino acid in the D- or L-optical isomer configuration; it is preferred that the peptides of this invention be assembled from amino-acid residues of the natural (L-) configuration.

Nomenclature used to define the peptides of Formulas I and II is that specified by the IUPAC [published in European Journal of Biochemistry, 138, 9-37 (1984)], wherein conventional representation of the peptides stipulates that in a peptide sequence the amino group appears to the left and the carboxyl group to the right.When the amino acid has enantiomeric forms, it is the L form of the amino acid which is represented unless otherwise stated. In the amino acid structural formulas, each residue is generally represented by a single or 3-letter designation, corresponding to the trivial name of the amino acid in accordance with the following list:

| TRIVIAL NAME | SYMBOL | ONE-LETTER SYMBOL |
|---|---|---|
| Alanine | Ala | A |
| Arginine | Arg | R |
| Asparagine | Asn | N |
| Aspartic Acid | Asp | D |
| Cysteine | Cys | C |
| Glutamine | Gln | Q |
| Glutamic Acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Unspecified Amino Acid | Xaa | X |

The group $^{125}$I-tyr indicates a radioactive mono-iodinated tyrosine residue.

## BRIEF DESCRIPTION OF DRAWING FIGURES

Fig. 1 is a schematic diagram of a general scheme for solid-phase synthesis of peptides of Formula I;

Fig. 2 is a plot of the competitive displacement of $^{125}$I-AP-III from a particulate rabbit lung membrane preparation by unlabelled AP-III and Peptide No. 9;

Fig. 3 is a plot of the competitive displacement of $^{125}$I-AP-III from particulate rabbit lung membrane preparation by unlabelled AP-III and Peptide No. 10;

Fig. 4 is a plot of the competitive displacement of $^{125}$I-Ap-III from particulate rabbit lung membrane preparation by unlabelled AP-III and Peptide No. 12;

Fig. 5 is a plot of the competitive displacement of $^{125}$I-AP-III from particulate rabbit lung membrane preparation by unlabelled by AP-III and peptide No. 11;

Fig. 6 is a time course of the variation of mean arterial pressure and renal excretion parameters when Peptide No. 1 is administered alone; and

Fig. 7 is a plot of the variation of mean arterial pressure and renal excretion parameters with co-infusion of Peptide No. 1 and AP-III.

## Detailed Description of the Invention

Peptides of Formula I can be synthesized by a suitable method, such as by exclusively solid-phase techniques, by partial solid-phase techniques, by fragment condensation or by classical solution addition. For example, the techniques of exclusively solid-phase peptide synthesis (SPPS) are set forth in the textbook "Solid-Phase Peptide Synthesis", Stewart & Young, 2nd Edition, Pierce Chemical Company, (1984).The fragment condensation method of synthesis is exemplified in U.S. Patent No. 3,972,859. Synthesis by the use of recombinant DNA techniques may also be used when no unnatural residues are present and should be understood to include the suitable employment of a structural gene coding for the desired form of analog. The synthetic peptide may be obtained by transforming a microorganism using an expression vector including a promoter and operator together with such structural gene and causing such

transformed microorganism to express the peptide. A non-human animal may also be used to produce the peptide by gene-farming using such a structural gene. The synthetic peptide is then suitably recovered from the animal by extraction from sera or the like.

The peptides are preferably prepared using solid phase synthesis, such as that described by Merrifield, J. Amer. Chem. Soc., 85, 2149 (1964), although other equivalent chemical syntheses known in the art can also be used as previously mentioned. Solid-phase synthesis is commenced from the carboxyl terminal end of the peptide by coupling a protected α-amino acid to a suitable resin. Such a starting material can be prepared by attaching α-amino protected amino acid residue to a suitably functionalized resin. See Fig. 1 which shows the general scheme for solid phase peptide synthesis.

Resins are commercially available from a variety of suppliers. The preparation of such a substituted resin is described by Stewart et al, "Solid Phase Peptide Synthesis".

Common to chemical preparation of the peptides of Formula I is the protection of the side chain groups of the various amino acid moieties with suitable protecting groups which will prevent a chemical reaction from occurring at that site until the group is ultimately removed. Usually also common is the protection of an alpha-amino protecting group to allow subsequent reaction to take place at that location. Accordingly, it is common that, as a step in the synthesis, an intermediate compound is produced which includes each of the amino-acid residues located in its desired sequence in the peptide chain with various of these residues linked to the side-chain protecting groups. In selecting a particular side chain protecting group to be used in the synthesis of the peptides, the following rules are followed: (a) the protecting group should be stable to the reagent and under the reaction conditions selected for removing the α-amino protecting group at each step of the synthesis, (b) the protecting group should retain its protecting properties and not be split off under coupling conditions and (c) the side chain protecting group must be removable, upon the completion of the synthesis containing the desired amino acid sequence, under reaction conditions that will not alter the peptide chain.Throughout the description herein, the term Ⓟ identifies a protecting group of the amino acid function, and (P') identifies a protecting group for a function on a side chain.

The α-amino protecting groups designated by Ⓟ are those known to be useful in the art in the step-wise synthesis of polypeptides. Among the classes of α-amino protecting groups represented by Ⓟ are (1) acyl-type protecting groups, such as formyl, trifluoroacetyl, phthalyl, p-toluenesulfonyl (Tos), benzenesulfonyl, nitrophenylsulfenyl, tritylsulfenyl, o-nitrophenoxyacetyl, chloroacetyl, acetyl, and γ-chlorobutyryl; (2) aromatic urethan-type protecting groups, such as benzyloxycarbonyl (Z) and substituted Z, such as p-chlorobenzyloxycarbonyl (CBZ), p-nitrobenzyloxycarbonyl, p-bromobenzyloxycarbonyl, p-methoxybenzyloxycarbonyl; (3) aliphatic urethan-type protecting groups, such as t-butyloxycarbonyl (Boc), diisopropylmethoxycarbonyl, isopropyloxycarbonyl, ethoxycarbonyl, allyloxycarbonyl; (4) cycloalkyl urethan-type protecting groups, such as fluorenylmethyloxycarbonyl (Fmoc), cyclopentyloxycarbonyl, adamantyloxycarbonyl, and cyclohexyloxycarbonyl; (5) thiourethantype protecting groups, such a phenylthiocarbonyl; (6) alkyl-type protecting groups, such as triphenylmethyl trityl), benzyl (Bzl); (7) trialkylsilane groups, such as trimethylsilane.The preferred α-amino protecting group is Boc.

When (P') is a protecting group for the hydroxyl group of Ser, (P') is preferably selected from the class consisting of acetyl (Ac), benzoyl (Bz), tert-butyl, trityl, tetrahydropyranyl, benzyl ether (Bzl), 2,6-dichlorobenzyl and Z. The most preferred protecting group is Bzl. (P') can be H, which means there is no protecting group on the hydroxyl group.

When (P') is a protecting group for Cys, (P') is preferably selected from the class consisting of p-methoxybenzyl (MeOBzl), p-methylbenzyl, thioethyl, acetamidomethyl, trityl and Bzl.The most preferred protecting group is acetamidomethyl. (P') can also be H, meaning that there is no protecting group on the sulfur.

When (P') is a protecting group for the guanidino group of arg, (P') is preferably selected from the class consisting of H, nitro, Tos, Z, adamantyloxycarbonyl and Boc. Tos is most preferred.

When (P') is H, or an ester-forming protecting group for the β-carboxyl group of Asp, (P') is preferably selected from the class consisting of Bzl, 2,6-dichlorobenzyl (Dcb), CBZ, methyl and ethyl. Bzl is most preferred.

When (P') is H, or a protecting group for the amido group of Gln or Asn, in (P') the latter case is preferably xanthyl (Xan).

The protecting group for carboxyl terminus is selected from the class consisting of OH, $OCH_3$, amides, hydrazides and esters, including an amide, a benzyl ester and a hydroxymethyl ester anchoring bond used in solid phase syntheses for linking to a solid resin support, such resin support exemplified by the following:
-NH-benzhydrylamine (BHA) resin support,
-NH-paramethylbenzhydrylamine (MBHA) resin support,
-NH-paramethoxybenzylhydryl-amine resin support,

-O-$CH_2$-polystyrene resin support,
-O-$CH_2$-benzyl-polystyrene resin support,
-O-$CH_2$-phenylacetamidomethyl-polystyrene resin support, and
-O-$CH_2$-phenyl-oxymethyl resin support.

The polystyrene polymer is preferably a copolymer of styrene with about 0.5 to 2% divinyl benzene as a cross-linking agent, such that the polystyrene polymer is completely insoluble in certain organic solvents.

The procedure leading to the growth of the peptide chain is exemplified herebelow for a procedure using the t-butoxycarbonyl group as the protective group for the amino function of the amino acid. See also the general scheme for SPPS in Fig. 1.

Following the coupling of the t-Boc protected amino acid to the resin support, the α-amino protecting group is removed, as by using trifluoroacetic acid (TFA) in methylene chloride ($CH_2Cl_2$), TFA alone or with HCl in dioxane. Preferably 50 volume % TFA in methylene chloride is used. The deprotection is carried out at a temperature between about 0°C and room temperature. Other standard cleaving reagents and conditions for removal of specific α-amino protecting groups may be used as described in Schroder & Lubke, "The Peptides", Academic Press, 1, 72-75 (1965).

After removal of the α-amino protecting group of the first α-amino acid, the remaining α-amino- and side chain-protected amino acids are coupled step-wise in the desired order to obtain the intermediate compound defined hereinbefore. As an alternative to adding each amino acid separately in the synthesis, some of the amino acids may be coupled to one another prior to addition to the solid phase reactor. The selection of an appropriate coupling reagent is within the skill of the art. Particularly suitable as coupling reagents are N,N′-dicyclohexylcarbodiimide (DCC) and N,N′-diisopropylcarbodiimide (DIC).

Activating reagents compatible with the routine operations of the solid phase synthesis of the peptides are well known in the peptide art. Examples of suitable activating reagents are: (1) carbodiimides, such as N,N′-diisopropyl carbodiimide, N-ethyl-N′-(3-dimethylaminopropyl)carbodiimide; (2) cyanamides such as N,N′-dibenzylcyanamide; (3) ketenimines; (4) isoxazolium salts, such as N-ethyl-5-phenyl isoxazolium-3′-sulfonate; (5) monocyclic nitrogen-containing heterocyclic amides of aromatic character containing one through four nitrogen atoms in the ring, such as imidazole, pyrazole, and 1,2,4-triazole derivatives. Specific heterocyclic amides that are useful include N,N′-carbonyl diimidazole, N,N′-carbonyl-di-1,2,4-triazole; (6) alkoxylated acetylene, such as ethoxyacetylene; (7) reagents which form a mixed anhydride with the carboxyl moiety of the amino acid, such as ethylchloroformate and isobutylchloroformate and (8) nitrogen-containing heterocyclic compounds having a hydroxy group on one ring nitrogen, such as N-hydroxyphthalimide, N-hydroxysuccinimide and 1-hydroxybenzotriazole (HOBt). Other activating reagents and their use in peptide coupling are described in Schroder & Lubke, supra, in Chapter III, (vide supra) and by Kapoor, J. Phar. Sci., 59, 1-27 (1970).

Each protected amino acid or amino-acid sequence is introduced into the solid phase reactor in about a two-to-ten fold excess, and the coupling is carried out in a medium of dimethylformamide (DMF):$CH_2Cl_2$ - (1:1) or in DMF or $CH_2Cl_2$ alone. In instances where the coupling is carried out manually, the success of the coupling reaction at each stage of the synthesis is monitored by the ninhydrin reaction, as described by E. Kaiser et al, Anal. Biochem., 34, 595 (1970). In cases wherein incomplete coupling occurs, the coupling procedure is repeated before removal of the α-amino protecting group prior to the coupling of the next amino acid. The coupling reactions can be performed automatically, as for example on Applied Biosystems Inc. model 430A peptide synthesizer.

After the desired amino-acid sequence has been completed, the intermediate peptide is removed from the resin support by treatment with a reagent, such as liquid hydrogen fluoride, which not only cleaves the peptide from the resin but also cleaves all remaining side chain protecting groups to obtain the peptide in its linear form.The cyclic form of the peptide is obtained by oxidization using iodine [as described by B. Kamber et al, Helvetica Chemica Acta., 63, 899-915 (1980)] or by air oxidation, or in accordance with other known procedures suitable for the considered type of protecting group for the sulfhydryl function of the cysteine.

As an alternative route, the intermediate peptide may be separated from the resin support by alcoholysis after which the recovered carboxyl terminal ester is converted to the acid by hydrolysis. Any side chain protecting groups may then be cleaved as previously described or by other known procedures, such as by catalytic reduction (e.g. Pd on $BaSO_4$). When using hydrogen fluoride for cleaving, anisole and 2-mercaptopyridine are included in the reaction vessel for scavenging. In cases where anhydrous HF is harmful for the peptide, a much more labile system can be used for the synthesis. Such systems are provided, among others, by other resins used with Bpoc, Fmoc, or Nps protected amino acids.

EXAMPLE I

Peptide No. 1 was prepared by solid phase synthesis on a phenylacetamidomethyl resin loaded with t-Boc-L-Tyr(BrZ) (substitution range: 0.6 to 0.8 mmol/g) that was purchased from Applied Biosystems Inc. The amino-acid derivatives used to assemble the peptide were chosen from the following list: t-Boc-L-Ser-(Bzl), t-Boc-L-Cys(Acm), t-Boc-L-Phe, t-Boc-Gly, t-Boc-L-Arg(Tos), t-Boc-L-Ile, t-Boc-L-Asp(OBzl), t-Boc-L-Leu, t-Boc-L-Asn (commercially available from Peptide International Inc. and/or Peninsula Laboratories and/or Applied Biosystems, Inc.). Characterization of the peptides include amino acids analysis (Beckman 6300HPA), sequencing (Applied Biosystem Protein Sequences 620) and FAB mass spectroscopy.

Synthesis Procedure

Peptide No. 1 was synthesized by automated machine synthesis (Applied Biosystems Inc. Model 430A Peptide Synthesizer) utilizing the standard protocols described in the Applied Biosystems Inc. System Software Version 1.30.

Cleavage and Deprotection Procedures

The dried, TFA deprotected peptide resin 1 g, 0.3-0.5 mmol/g), anisole (1 g) and 2-mercaptopyridine (0.4 g) were stirred at 0° C in liquid HF (10-15 ml) in a standard Teflon vessel for $1\frac{1}{2}$ hours.The HF was evaporated and the residue was triturated in diethylether (4 x 40 ml), ethyl acetate (4 x 40 ml), and extracted with 25%-80% acetic acid (AcOH) (3 x 30 ml) by filtration.The filtrate was concentrated to 5 ml volume with a rotary evaporator at 5-10° C @ 1 mm Hg. The residue was redissolved in water containing 0.05% TFA and lyophylized.

Cyclization Procedure

To a magnetically stirred solution of 200 mg iodine in 200 ml of a 1:4 water:acetic acid mixture was added an aliquot of 200 mg of the material obtained in the previous step. After 1 to 6 hours of stirring at 25° C, 200 ml of deionized water was added and the resulting mixture was extracted twice with 500 ml chloroform and once with 500 ml diethylether. The aqueous phase was concentrated to a volume of 10 ml with a rotary evaporator at 5-10° C @ 1 mm Hg. 15The residue was redissolved in 50 ml of water containing 0.05% TFA for purification.

Purification Procedure

The above solution was filtered through a Whatman 934 AH glass fiber filter and was applied to a chromatography column (50 mm x 300 mm) packed with 200 g of Vydac C18 silica (Vydac 218TPB 15-20; 30 mμ pore size).The column was eluted at a flow of 8 ml/min., first with 5% acetonitrile in water containing 0.05% TFA followed by a 2 hour gradient from 5 to 40% acetonitrile in water containing 0.05% TFA while collecting fractions.The product usually eluted after 1 hour of the gradient as indicated by monitoring the fractions by Sakaguchi reagent (G. Borin et al, Int. J. Pep. Prot. Res., 10, 27-38 (1977). The peptide-containing fractions were analyzed by analytical HPLC. ThoSe fractions determined to be greater than 85% pure were combined. The peptide was obtained in homogeneous form by semi-preparative HPLC on a Vydac C18 column (218TP510, i.d. 10mm, length 25 cm, 5 micron particle size) using a linear gradient of 15 to 35% acetonitrile in 0.05% trifluoroacetic acid at a flow rate of 4 ml/min. Homogeneity of the collected fractions was determined by analytical HPLC.

Peptide No. 1

Peptide No. 1, having the formula

ser-ser-
cys-gly-arg-ile-asp-arg-ile-gly-ser-gly-leu-gly-cys-asn-
|__________________________________________|
ser-phe-arg-tyr,

was synthesized and isolated using the procedure described above. Its structure was confirmed by:
- amino acid analysis (theoretical, found): Asp, Asn (2, 2.05); Ser (4, 3.19), Gly (4, 3.90), Ile (2, 1.77), Leu (1, 1.05), Tyr (1, 0.79), Phe (1, 1.0), Arg (3, 2.67).
- FAB mass spectroscopy: the isotope pattern (M+H) cluster is measured at expected theoretical values 2146/2147.

Peptide No. 2 through No. 12

Peptide Nos. 2-12 were prepared by solid phase synthesis on a 4-methylbenzhydrylamine resin (p-MBHA, 1% cross-linked polystyrene/divinylbenzene, 200-400 mesh) substituted with 0.3 to 0.5 meq/g of t-Boc-L-Arg(Tos). The amino acid derivatives 25utilized to assemble the peptide chain include: t-Boc-Ser-(Bzl), t-Boc-Cys(Acm), t-Boc-Phe, t-Boc-Gly, t-Boc-Arg(Tos), t-Boc-Ile, t-Boc-Asp (OBzl), t-Boc-Ala, t-Boc-Gln(Xan), t-Boc-Gln, t-Boc-Leu, t-Boc-Asn, t-Boc-Asn(Xan) commercially available from Peptide International Inc. and/or Peninsula Laboratories and/or Applied Biosystems Inc. All reagents and solvents were ACS reagent grade or conformed to USP standard defined in the U.S. Pharmacopeia. The deprotection, cyclization and purfication procedures were similar generally to the procedures described above for Peptide No. 1.

Synthesis Procedure

The peptides were synthesized by automated machine synthesis or by manual synthesis. In the case of automated machine synthesis, an Applied Biosystems Inc. Peptide Synthesizer (model 430A) was used and the synthetic steps were performed according to the standard protocols described in the Applied Biosystems Inc. System Software Version 1.30.

In the case of the manual synthesis, the peptide chain was assembled by a procedure based on repetition of the following three operations (multiple couplings were performed when necessary):

| Operation | Step | Reagents | No. Times | Mix Time (min.) |
|---|---|---|---|---|
| I | | DEPROTECTION | | |
| | 1 | $CH_2Cl_2$* | 3 | 1 |
| | 2 | Prewash w/50% trifluoroacetic acid (TFA)/$CH_2Cl_2$ | 1 | 1 |
| | 3 | Deprotect w/50% TFA/$CH_2Cl_2$ | 1 | 30 |
| | 4 | $CH_2Cl_2$ | 3 | 1 |
| | 5 | Isopropanol | 2 | 1 |
| | 6 | $CH_2Cl_2$ | 5 | 1 |
| | 7 | Kaiser Test | | |
| Operation | Step | Reagents | No. Times | Mix Time (min.) |
| II | | NEUTRALIZATION | | |
| | 1 | 5% diisopropylethylamine/$CH_2Cl_2$ | 2 | 2 |
| | 2 | $CH_2Cl_2$ | 3 | 1 |
| III | | COUPLING | | |
| | 1 | swell resin with $CH_2Cl_2$/DMF; | 3 | 1 |
| | 2 | Boc-aa (4-10 eq.)/$CH_2Cl_2$/DMF; | 3 | 0.5 |
| | 3 | 4.0 eq. of 1-M DCC/$CH_2Cl_2$ | 1 | 120 |
| | 4 | $CH_2Cl_2$ | 2 | 1 |
| | 5 | Ethanol | 1 | 1 |
| | 6 | $CH_2Cl_2$ | 3 | 1 |
| (See General Scheme in Fig. 1 for Peptide Synthesis.) | | | | |

Peptide No. 2

Peptide No. 2 having the formula

**cys-gly-arg-ile-asp-arg-ile-gly-ser-gly-leu-gly-cys-$NH_2$**

was synthesized using the automated procedure as described in the peptide synthesis section. FAB Mass Spectroscopy: the isotope pattern (M + H) cluster is measured at the expected value 1304/1305.

Amino acid analysis (Theoretical, found):

asp (1, 1.04), ser (1, 0.92), gly (4, 3.81), ile (2, 1.86), leu (1, 1.03), arg (2, 2.14).

Peptide No. 3

Peptide No. 3 having the formula

**ser-ser-**

**cys-gly-arg-ile-asp-arg-ile-gln-ser-gly-leu-gly-cys-asn-**

**ser-phe-arg-$NH_2$**

was synthesized using the manual procedure as described in the peptide synthesis section, above. Sequence analysis shows that the peptide with the correct sequence was isolated.

Amino acid analysis (Theoretical, found): asp, asn (2, 2.16), ser (4, 3.59), gln (1, .03), gly (3, 3.12), ile (2, 1.91), leu (1, 1.07), phe (1, 1.02), arg (3, 3.09).

Peptide No. 4

Peptide No. 4 having the formula

ser-ser-
cys-gly-arg-ile-asp-arg-ile-gly-ala-gln-leu-gly-cys-asn-
|_______________________________________________|
ser-phe-arg-$NH_2$

was synthesized using the manual procedure as described in the peptide synthesis section. Sequence analysis shows that the peptide with the correct sequence was isolated.

Amino acid analysis (Theoretical, found): asp, asn (2, 1.93), ser (3, 2.09), gln (1, 1.00), gly (3, 2.93), ile (2, 1.77), leu (1, 0.98), Phe (1, 0.93), arg (3, 2.70).

Peptide No. 5

Peptide No. 5 having the formula

ser-ser-
cys-gly-arg-ile-asp-arg-ile-gly-ala-gln-ser-gly-cys-asn-
|_______________________________________________|
ser-phe-arg-$NH_2$

was synthesized using the manual procedure as described in the peptide synthesis section, above. Sequence analysis shows that the peptide with the correct sequence was isolated.

Amino acid analysis (Theoretical, found): asp, asn (2, 2.24), ser (4, 3.63), gln (1, 1.10), gly (3, 3.23), ala (1, 1.17), ile (2, 1.87), phe (1, 1.01), arg (3, 2.73).

Peptide No. 6

Peptide No. 6 having the formula

ser-ser-
cys-gly-arg-ile-asp-arg-ile-gln-leu-gly-cys-asn-ser-phe-
|_______________________________________|
arg-$NH_2$

was synthesized using the manual procedure as described in the peptide synthesis section, above.

Amino acid analysis (Theoretical, found): asp, asn (2, 2.20), ser (3, 2.35), gln (1, 1.00), gly (2, 2.06), ile (2, 1.70), leu (1, 1.02), phe (1, 1.00), arg (3, 2.62).

Peptide No. 7

Peptide No. 7 having the formula

```
                                                   ser-ser-
cys-gly-arg-ile-asp-arg-ile-gly-ser-gly-cys-asn-ser-phe-
|___________________________________________________|
arg-NH2
```

was synthesized using the manual procedure as described in the peptide synthesis section, above.

Amino acid analysis (Theoretical, found):
asp, asn (2, 2.19), ser (4, 3.47), gly (3, 3.13), ile (2, 1.76, phe (1, 1.00), arg (3, 2.64).

<u>Peptide No. 8</u>

Peptide No. 8 having the formula

```
                                                   ser-ser-
cys-gly-arg-ile-asp-arg-ile-gly-ala-gly-cys-asn-ser-phe-
|___________________________________________________|
arg-NH2
```

was synthesized using the manual procedure as described in the peptide synthesis section, above.

Amino acid analysis (Theoretical, found):
asp, asn (2, 1.98), ser (2, 1.71), gly (4, 3.87), ile (2, 1.68), leu (1, 1.03), phe (1, 1.00), arg (3, 2.64).

<u>Peptide No. 9</u>

Peptide No. 9 having the formula

```
                                                   ser-ser-
cys-gly-arg-ile-asp-arg-ile-gly-ser-gly-leu-gly-cys-asn-
|__________________________________________________________|
ser-phe-arg-NH2
```

was synthesized using the manual procedure as described in the peptide synthesis section, above. Sequence analysis shows that the peptide with the correct sequence was isolated.

Amino acid analysis (Theoretical, found):
asp, asn (2, 2.09), ser (4, 3.61), gly (4, 4.39), ile (2, 1.82), leu (1, 1.00), phe (1, 0.96), arg (3, 3.14).

<u>Peptide No. 10</u>

Peptide No. 10 having the formula

```
                                                    ser-ser-
cys-gly-arg-ile-asp-arg-ile-gly-ala-gly-leu-gly-cys-asn-
|__________________________________________________________|
ser-phe-arg-NH2
```

was synthesized using the manual procedure as described in the peptide synthesis section, above. Sequence analysis shows that the peptide with the correct sequence was isolated.

Amino acid analysis (Theoretical, found):
asp, asn (2, 2.19), ser (3, 2.75), gly (4, 4.24), ala (1, 1.26), ile (2, 1.87), leu (1, 1.06), phe (1, 1.01), arg (3, 2.55).

Peptide No. 11

Peptide No. 11 having the formula

```
cys-gly-arg-ile-asp-arg-ile-gly-ser-gly-leu-gly-cys-
 |_________________________________________________|
asn-ser-phe-arg-NH2
```

was synthesized using the manual procedure as described in the peptide synthesis section, above.

Amino acid analysis (Theoretical, found):
asp, asn (2, 1.98), ser (2, 1.71), gly (4, 3.87), ile (2, 1.68), leu (1, 1.03), phe (1, 1.00), arg (3, 2.64).

Peptide No. 12

Peptide No. 12 having the formula

```
                                                 ser-ser-
cys-gly-arg-ile-asp-arg-ile-gly-leu-gly-cys-asn-ser-phe-
 |__________________________________________|
arg-NH2
```

was synthesized using the manual procedure as described in the peptide synthesis section, above.

Amino acid analysis (Theoretical, found):
asp, asn (2, 2.04), ser (3, 2.71), gly (3, 2.93), ile (2, 1.86), leu (1, 1.03), phe (1, 1.00), arg (3, 2.71).

## BIOLOGICAL EVALUATION

Peptide Nos. 1 through 12 were evaluated for their biological activity.The biological assays used to evaluate the compounds are listed below and then described in greater detail thereafter:

A. Competitive binding using a particulate rabbit lung membrane preparation;

B. c-GMP production in isolated rat aorta;

C. Effects on mean arterial blood pressure, urinary flow rate and urinary sodium excretion in conscious rats.

### A.Competitive Binding Experiments on Isolated Rabbit Lung Membrane.

Preparation of rabbit lung membranes -

The procedure was carried out at 4° C. Frozen rabbit lung lobes (Pel-Freez, Rogers, AR) were minced and then homogenized for 30 sec. with a Brinkmann Polytron in 5 volumes of a solution containing 0.25 M sucrose, 3 mM $MgCl_2$, 1 mM EDTA and 5 mM Tris pH 7.5. The homogenate was filtered through

cheesecloth to remove some fat and connective tissue and the filtrate was centrifuged at 5,000 x g for 20 min. The supernatant was centrifuged at 100,000 x g for 90 min. The washed pellet was suspended in 50 mM Tris pH 7.5 at a final concentration of 4 mg/ml.The Biorad protein assay kit was used to assay membrane protein. The rabbit lung membrane preparation was stored in aliquots at -80 °C and was stable for at least 2 months. The binding assay utilized 0.25 ml of a solution containing 50 mM Tris pH 7.5, 0.1% bovine serum albumin, 100 $\mu$g membrane protein and $^{125}$I-AP-III (2-3 x $10^4$cpm; specific activity of about 740 Ci/mmol) in the absence or presence of unlabelled peptide.The reaction was initiated by the addition of membranes and the mixture was incubated at 25 °C for 30 min. The incubation was terminated with 4 ml ice-cold 50 mM Tris pH 7.5 and the mixture was filtered to separate membrane-bound labelled peptide from free ligand using a PHD Cell Harvester (Cambridge Technology, Inc., Mass.). The incubation tube and filter were washed with ice-cold buffer. Filters were assayed for radioactivity in a Micromedic gamma counter. Nonspecific binding was defined as binding in the presence of $10^{-6}$M unlabelled AP-III. Specific binding was calculated as total binding minus nonspecific binding. Binding data were analyzed by a nonlinear least-squares curve fitting program.

It was previously found that the rabbit lung contains a large number of specific binding sites for AP-III, presumably consisting of a single class of high affinity receptors, as indicated by a linear Scatchard plot [G.M. Olins et al, Biochem. Biophys. Res. Commun., 140, 302-307 (1987)]. However, it has now been discovered that the peptides of this invention selectively bind to a subset of the total atrial peptide binding sites.The results obtained using the selective peptides of the invention suggest that Ap-III is a non-selective ligand which binds to at least two binding sites with equal affinity. From the competition binding experiments, the selective peptides of Formula I only bind to approximately 73% of these sites as shown in Figs. 2 to 5. Thus, there appear to be two classes of atrial peptide binding sites in a ratio of 3:1. Similar results were obtained with membranes prepared from fresh or frozen lung tissue.

The data are reported in Table II. In addition to absolute inhibition constants ($K_i$ in nM) that provide a quantitative measurement for affinity to the AP receptors, the selectivity factor (defined as the ratio of the $K_i$ for the vasorelaxant site to the $K_i$ for the non-vasorelaxant site) is provided for each compound.

Figs. 2 to 5 show competitive binding between radioiodinated AP-III and peptides of the invention to rabbit lung membranes.Figs. 2 to 5 present the binding data as a plot of the ratio B/Bo versus the negative logarithm of the molar concentration of unlabelled peptides.The ratio B to Bo expresses the ratio of specific binding of the radiolabelled ligand in the absence (Bo) or in the presence (B) of various concentrations of the unlabelled peptides.The membranes were equilibrated with 70 pM $^{125}$I-AP-III and varying concentrations of unlabelled peptides of the invention: $^{125}$I-AP-III vs. AP-III; vs. Peptide No. 9 (Fig. 2); vs. Peptide No. 10 (Fig. 3); vs. Peptide No. 12 (Fig. 4); and vs. Peptide No. 11 (Fig. 5).

## TABLE II

Table II is a comparison of the inhibition constants ($K_i$ in nM) of the selective peptides of the invention for the atrial peptide guanyl cyclase coupled and non-guanyl cyclase coupled receptor sites for rabbit lung membranes.

| No. | $K_i$ of AP24 (nM) | $K_i$ of the uncoupled site (nM) | Relative Potency* | $K_i$ of the coupled site (nM) | Relative Potency | Selectivity Factor** coupled vs. non-coupled |
|---|---|---|---|---|---|---|
| 1 | 0.3 | 1.5 | 0.32 | >100 | <0.003 | > 66 |
| 9 | 0.32 | 0.1 | 3.2 | >100 | <0.003 | >1000 |
| 11 | 0.2 | 0.11 | 1.8 | >100 | <0.003 | > 909 |
| 2 | 0.16 | 0.87 | 0.19 | >100 | <0.003 | > 115 |
| 3 | 0.15 | 0.96 | 0.16 | >100 | <0.003 | > 104 |
| 10 | 0.26 | 0.082 | 3.2 | >100 | <0.003 | >1220 |
| 4 | 0.26 | 0.39 | 0.67 | >100 | <0.003 | > 250 |
| 5 | 0.32 | 1.0 | 0.32 | >100 | <0.003 | > 100 |
| 6 | 0.18 | 1.2 | 0.15 | >100 | <0.003 | > 83 |
| 7 | 0.18 | 0.11 | 1.6 | 52 | 0.0035 | 472 |
| 8 | 0.25 | 0.31 | 0.81 | >100 | <0.003 | > 322 |
| 12 | 0.26 | 0.14 | 1.9 | >100 | <0.003 | > 714 |
| 13 | 0.25 | 1.6 | 0.16 | >100 | <0.003 | > 62 |
| 14 | 0.2 | 9.8 | 0.02 | >100 | <0.003 | > 10 |
| 15 | 0.26 | 0.35 | 0.74 | 75 | 0.0035 | > 214 |

* Relative potencies are determined using AP-III as standard (given an arbitrary value of 1.0).

** Selectivity Factor is the ratio of the $K_i$ for the vasorelaxant (guanyl cyclase-coupled) receptor site to the $K_i$ for the non-vasorelaxant (guanyl cyclase-uncoupled) receptor site for each considered compound.

B - Effect of Peptide No. 1 on cGMP Production in Isolated Rat Aorta

Isolated rat aorta segments were incubated with AP-III (20 nM) under conditions which result in a maximal response in the production of cGMP. Peptide No. 1, which binds selectively to the uncoupled AP

binding site, was incubated under conditions identical to the control at a concentration of 700 nM. The results from duplicate experiments using three different aorta segments are shown in Table III.There was little or no stimulation of guanylate cyclase activity, under conditions which produced an average 4.5 fold increase in cGMP in the presence of AP-III.

The following protocol was used to generate cyclic GMP in rat aortic tissue. The cGMP levels were measured using a radioimmunoassay kit. The thoracic aorta of male Sprague-Dawley rats was surgically removed and placed in a beaker of oxygenated Krebs-Bicarbonate Buffer, pH 7.4. The tissue was cleaned of fat and blood and each aorta was cut into two 10 mm segments. Each of these 10 mm segments was longitudinally split, thus yielding four segments per thoracic aorta.The segments of aorta, each weighing approximately 600-800 micrograms, were preincubated for 30 minutes in a small petri dish containing the following:

3 ml oxygenated Krebs-Bicarbonate buffer
$2 \times 10^{-5}$ mM Norepinephrine
0.1 mM IBMX (Isobutylmethylxanthine)

All incubations were done in a Forma Incubator at 37° C under atmosphere of 95% $O_2$ and 5% $CO_2$. After the pre-incubation, 20 nM AP-III was added to the appropriate segment, the test peptide of the invention was added to the companion segments. For each rat, one of the four segments was used as the Control, one segment was used for the Standard 20 nM AP-III and two segments were used for the test peptide of Formula I.

The incubation period for the stimulation of cyclic GMP in the presence of AP-III was 30 minutes.

Following the incubation, the tissue segments were removed and frozen on dry-ice, the segments were then weighed and placed in test tubes for extraction with absolute ethanol. The tissues were homogenized with the Polytron and then centrifuged at 3000 rpm for 15 minutes at 4° C. The supernatant was poured off into another set of tubes and evaporated to dryness in a vacuum oven. After the tubes were dry, the samples were taken up in 2 ml 0.05 M sodium acetate buffer, pH 6.2. One hundred microliters of each extract was used for assay in the radioimmunoassay (NEN RIA Kit, $^{125}I$ tracer).

TABLE III

| Effect of Peptide No. 1 on cGMP Levels in Isolated Rat Aorta | | |
|---|---|---|
| Rat Aorta | Treatment | cGMP Produced (pmol/gww) |
| A911 | Control | 1.32 |
| | AP III (20 nM) | 11.54 |
| | Peptide #1 (700 nM) | 1.66 |
| | Peptide #1 (700 nM) | <0.56 |
| B911 | Control | 1.19 |
| | AP III (20 nM) | 5.67 |
| | Peptide #1 (700 nM) | 1.23 |
| | Peptide #1 (700 nM) | 2.59 |
| A918 | Control | 5.75 |
| | AP III (20 nM) | 19.72 |
| | Peptide #1 (700 nM) | 3.36 |
| | Peptide #1 (700 nM) | 10.92 |

C. - Effects on Mean Arteral Pressure, Urine Flow Rate, and Urinary Sodium Excretion

Peptide No. 1 which binds selectively to the guanylcyclase-free receptor of AP produces no increase of cyclic GMP production.Since many studies have implied that increases in cyclic GMP levels mediate the action of atrial peptide [R.J. Winquist. et al, Proc. Nat'l. Acad. Sci. USA, 81, 17661-64 (1984); M. Sat et al, Hypertension, 8, 762-71 (1986)], studies in conscious rats infused with peptide of the invention were conducted to study the effect of this peptide on the endogeneous level of atriopeptins (by monitoring urine flow rate and urinary sodium excretion) and on the potentiation and prolongation of the duration of action of

AP-III.

Under methohexital anesthesia, catheters were implanted in the jugular vein, carotid artery, and urinary bladder. Two to four hours after surgery in conscious rats, a saline infusion was begun at 30 ul/min and continued at the same rate for the duration of the experiment. A one hour equilibration and stabilization period was followed by six 20-minute experimental collection periods in Table IV. The first collection was a control period (C1), the next three were experimental compound infusion periods E1, E2, E3) and the last two were recovery periods ($R_1$, $R_2$). Four groups were studied. Group I was infused with a Control peptide that does not bind to AP receptors and has no known biological response. This group served as a time-control for the other groups.Group II was infused with Peptide No. 1 during E1, E2 and E3. Group III was infused with AP-III during E1, E2 and E3. Group IV received Peptide No. 1 from time zero to the end of the experiment, and received AP-III during E1, E2 and E3.

TABLE IV

| | | | C1 | E1 | E2 | E3 | R1 | R2 |
|---|---|---|---|---|---|---|---|---|
| Time (min) | 0 | 60 | 80 | 100 | 120 | 140 | 160 | 180 |

I. | Time-Control | (80–140)
Control Peptide

II. | Peptide No. 1 | (80–140)
16.0 µg/kg/min

III. | AP-III | (80–140)
0.05 µg/kg/min

IV. | Peptide No. 1 |
16.0 µg/kg/min

| AP-III |
0.05 µg/kg/min

Fig. 6 shows that mean arterial pressure, urine flow rate, and urinary sodium excretion were stable in the time-control group. Peptide No. 1 had no effect on mean arterial pressure when infused alone, but produced a mild diuresis and natriuresis. Fig. 7 shows that Peptide No. 1 potentiated the depressor (but not the diuretic or natriuretic) response to AP-III. Pharmacological blockade of guanyl cyclase-free receptors with Peptide No. 1 has a small effect on baseline levels of urine flow rate and urinary sodium excretion, and potentiates the mean arterial pressure.

Peptides of Formula I have been demonstrated to have a high affinity associated with high selectivity for one single type of receptor site of AP-III. This particular type of receptor site could be hypothesized to affect the level of free atriopeptides in plasma. The *in vivo* effects found with the peptides of Formula I relate to interaction with this particular type of receptor site.

Although this invention has been described with respect to specific embodiments, the details. of these embodiments are not to be construed as limitations. Various equivalents, changes and modifications may be made without departing from the spirit and scope of this invention, and it is understood that such equivalent embodiments are part of this invention.

## Claims

1. A peptide of the formula

$$\text{A-}X^1_m\text{-cys-gly-arg-ile-asp-arg-ile-}X^2_n\text{-gly-cys-}X^3_p\text{-B}$$

wherein
$X^1$ is a fragment consisting of one to about eight amino-acid residues selected from arg, ser, pro and leu, and wherein m is an integer selected from zero through four;
$X^2$ is a fragment consisting of one to about six amino-acid residues selected from gly, leu, ser, ala and gln, and wherein n is an integer selected from zero through three;
$X^3$ is a fragment consisting of one to about six amino-acid residues selected from lys, asn, ser, phe, arg, tyr and $^{125}$I-tyr, wherein p is an integer selected from zero through four;
wherein A represents an amino terminus or its pharmaceutically-acceptable salt and wherein B represents a carboxyl terminus or its pharmaceutically-acceptable ester, amide or salt;
with the proviso that the sum of all amino-acid residues in said peptide is an integer in a range from about 13 through about 25.

2. Peptide of Claim 1 wherein $X^1$ is a fragment consisting of one to eight amino-acid residues and m is zero or one.

3. Peptide of Claim 2 wherein $X^1$ is a peptidic fragment consisting of four or six amino-acid residues selected from ser, arg and leu.

4. Peptide of Claim 3 wherein $X^1$ is a fragment consisting of two to four amino-acid residues selected from ser and arg.

5. Peptide of Claim 4 wherein $X^1$ is a fragment consisting of one or two serine residues.

6. Peptide of Claim 1 wherein m is zero.

7. Peptide of Claim 1 wherein $X^2$ is a fragment consisting of two to six amino-acid residues and n is one.

8. Peptide of Claim 7 wherein $X^2$ is a peptidic fragment consisting of two or four amino-acid residues.

9. Peptide of Claim 8 wherein $X^2$ is selected from
gly-ala-gly-leu,
gly-ala-gln-leu,
gln-ser-gly-leu,
gly-ala-gln-ser,
gln-leu,
gly-ser and
gly-ala.

10. Peptide of Claim 8 wherein $X^2$ is a fragment consisting of four amino-acid residues.

11. Peptide of Claim 10 wherein $X^2$ is selected from
gly-ser-gly-leu and
gly-ala-gly-leu.

12. Peptide of Claim 8 wherein $X^2$ is a fragment consisting of two amino-acid residues.

13. Peptide of Claim 12 wherein $X^2$ is gly-leu.

14. Peptide of Claim 1 wherein n is one.

15. Peptide of Claim 1 wherein $X^3$ is a peptidic fragment consisting of one to six amino-acid residues and p is zero or one.

16. Peptide of Claim 15 wherein $X^3$ is a fragment consisting of four or five amino-acid residues.

17. Peptide of Claim 16 wherein $X^3$ is asn-ser-phe-arg-tyr.

18. Peptide of Claim 16 wherein $X^3$ is a fragment consisting of four amino-acid residues selected from asn, ser, phe and arg.

19. Peptide of Claim 18 wherein $X^3$ is asn-ser-phe-arg.

20. Peptide of Claim 1 wherein r is zero.

21. Peptide of Claim I wherein
$X^1$ is selected from
arg-arg
arg-ser and

ser-ser;
$X^2$ is selected from
gly-ser-gly-leu,
gly-ala-gly-leu,
gln-ser-gly-leu,
gly-ala-gln-ser,
gly-ala-gln-leu,
gly-ser,
gln-leu,
gly-ala and
gly-leu;
$X^3$ is selected from
asn-ser-phe,
asn-ser-phe-arg,
lys-ser-phe-arg,
asn-ser-phe-arg-tyr,
lys-ser-phe-arg-tyr,
asn-ser-phe-arg-$^{125}$I-tyr and
lys-ser-phe-arg-$^{125}$I-tyr.
and wherein m is zero or one, n is one and p is zero or one.

22. Peptide of Claim 21 wherein
$X^1$ is selected from
arg-arg,
arg-ser and
ser-ser;
$X^2$ is selected from
gly-ser-gly-leu,zf gly-ser,
gln-leu and
gly-ala;
$X^3$ is selected from
asn-ser-phe-arg,
lys-ser-phe-arg,
asn-ser-phe-arg-tyr,
lys-ser-phe-arg-tyr,
asn-ser-phe-arg-$^{125}$I-tyr and
lys-ser-phe-arg-$^{125}$I-tyr.

23. Peptide of Claim 22 wherein $X^1$ is selected from
arg-arg,
arg-ser and
ser-ser;
$X^2$ is gly-ser-gly-leu;
$X^3$ is selected from
asn-ser-phe-arg-tyr,
lys-ser-phe-arg-tyr,
asn-ser-phe-arg-$^{125}$I-tyr and
lys-ser-phe-arg-$^{125}$I-tyr.

24. Peptide of Claim 23 which is

**cys-gly-arg-ile-asp-arg-ile-gly-ser-gly-leu-gly-cys-$NH_2$.**

25. Peptide of Claim 23 which is

ser-ser-cys-gly-arg-ile-asp-arg-ile-gly-ser-gly-leu-gly-cys-asn-
ser-phe-arg-tyr.

26. Peptide of Claim 22 wherein $X^1$ is selected from
arg-arg,
arg-ser and
ser-ser;
$X^2$ is selected from
gly-ser,
gly-leu,
gln-leu and
gly-ala;
$X^3$ is selected from
asn-ser-phe-arg,
lys-ser-phe-arg,
asn-ser-phe-arg-tyr,
lys-ser-phe-arg-tyr,
asn-ser-phe-arg-$^{125}$I-tyr and
lys-ser-phe-arg-$^{125}$I-tyr.

27. Peptide of Claim 26 wherein said B terminus is a primary amide moiety or a lower alkyl secondary or tertiary amide moiety when $X^3$ is selected from
asn-ser-phe-arg and
lys-ser-phe-arg.

28. Peptide of Claim 22 wherein $X^1$ is selected from
arg-arg,
arg-ser and
ser-ser;
$X^2$ is gly-ser-gly-leu;
$X^3$ is selected from
asn-ser-phe-arg and
lys-ser-phe-arg.

29. Peptide of Claim 28 which is

cys-gly-arg-ile-asp-arg-ile-gly-ser-gly-leu-gly-cys-asn-ser-phe-
arg-$NH_2$.

30. Peptide of Claim 21 wherein
$X^1$ is selected from
arg-arg,
arg-ser and
ser-ser;
$X^2$ is selected from
gly-ser-gly-leu,
gly-ala-gly-leu,
gln-ser-gly-leu,
gly-ala-gln-ser,
gly-ala-gln-leu,
gly-leu,
gly-ala,
gly-ser and
gln-leu;
$X^3$ is selected from

asn-ser-phe-arg and
lys-ser-phe-arg;
and wherein each of m, n and p is one.

31. Peptide of Claim 30 wherein $X^1$ is ser-ser;
$X^2$ is selected from
gly-ala-gly-leu,
gly-ser-gly-leu,
gln-ser-gly-leu,
gly-ala-gln-ser and
gly-ala-gln-leu;
$X^3$ is asn-ser-phe-arg.

32. Peptide of Claim 31 which is

ser-ser-cys-gly-arg-ile-asp-arg-ile-gln-ser-gly-leu-gly-cys-asn-ser-
phe-arg-$NH_2$.

33. Peptide of Claim 31 which is

ser-ser-cys-gly-arg-ile-asp-arg-ile-gly-ala-gln-ser-gly-cys-asn-ser-
phe-arg-$NH_2$.

34. Peptide of Claim 31 which is

ser-ser-cys-gly-arg-ile-asp-arg-ile-gly-ala-gln-leu-gly-cys-asn-ser-
phe-arg-$NH_2$.

35. Peptide of Claim 31 which is

ser-ser-cys-gly-arg-ile-asp-arg-ile-gly-ser-gly-leu-gly-cys-asn-ser-
phe-arg-$NH_2$.

36. Peptide of Claim 31 which is

ser-ser-cys-gly-arg-ile-asp-arg-ile-gly-ala-gly-leu-gly-cys-asn-ser-
phe-arg-$NH_2$.

37. Peptide of Claim 30 wherein
$X^1$ is selected from
arg-arg,
arg-ser and
ser-ser;
$X^2$ is selected from
gln-leu,
gly-ser,
gly-ala and
gly-leu;
$X^3$ is selected from
asn-ser-phe-arg and
lys-ser-phe-arg;
and wherein each of m, n and p is one.

38. Peptide of Claim 37 wherein $X^1$ is ser-ser;
$X^2$ is selected from
gln-leu,
gly-ser,
gln-ala and
gly-leu;
$X^3$ is selected from
asn-ser-phe-arg and
lys-ser-Phe-arg.

39. Peptide of Claim 38 which is

ser-ser-cys-gly-arg-ile-asp-arg-ile-gln-leu-gly-cys-asn-ser-phe-arg-$NH_2$.

40. Peptide of Claim 38 which is

ser-ser-cys-gly-arg-ile-asp-arg-ile-gly-ser-gly-cys-asn-ser-phe-arg-$NH_2$.

41. Peptide of Claim 38 which is

ser-ser-cys-gly-arg-ile-asp-arg-ile-gly-ala-gly-cys-asn-ser-phe-arg-$NH_2$.

42. Peptide of Claim 38 which is

ser-ser-cys-gly-arg-ile-asp-arg-ile-gly-leu-gly-cys-asn-ser-phe-arg-$NH_2$.

43. Use of a therapeutically-effective amount of a peptide of the formula

A-$X^1_m$-cys-gly-arg-ile-asp-arg-ile-$X^2_n$-gly-cys-$X^3_p$-B

wherein
$X^1$ is a fragment consisting of one to about eight amino-acid residues selected from arg, ser, pro and leu, and wherein m is an integer selected from zero through four;
$X^2$ is a fragment consisting of one to about six amino-acid residues selected from gly, leu, ser, ala and gln, and wherein n is an integer selected from zero through three;
$X^3$ is a fragment consisting of one to about six amino-acid residues selected from lys, asn, ser, phe, arg, tyr and $^{125}$I-tyr, wherein p is an integer selected from zero through four;
wherein A represents an amino terminus or its pharmaceutically-acceptable salt and wherein B represents a carboxyl terminus or its pharmaceutically-acceptable ester, amide or salt;
with the proviso that the sum of all amino-acid residues in said peptide is an integer in a range from about 13 through about 25, for the manufacture of a medicament for treating hypertension.

44. Use according to Claim 43 wherein $X^1$ of the peptide is a fragment consisting of one to eight amino-acid residues and m is zero or one.

45. Use according to Claim 44 wherein $X^1$ is a peptidic fragment consisting of four or six amino-acid residues selected from ser, arg and leu.

46. Use according to Claim 45 wherein $X^1$ is a fragment consisting of two to four amino-acid residues selected from ser and arg.

47. Use according to Claim 46 wherein $X^1$ is a fragment consisting of one or two serine residues.

48. Use according to Claim 43 wherein m is zero.

49. Use according to Claim 43 wherein $X^2$ is a fragment consisting of two to six amino-acid residues and n is one.

50. Use according to Claim 49 wherein $X^2$ is a peptidic fragment consisting of two or four amino-acid residues.

51. Use according to Claim 50 wherein $X^2$ is selected from
gly-ala-gly-leu,
gly-ala-gln-leu,
gln-ser-gly-leu,
gly-ala-gln-ser,
gln-leu,
gly-ser and
gly-ala.

52. Use according to Claim 49 wherein $X^2$ is a fragment consisting of four amino-acid residues.

53. Use according to Claim 52 wherein $X^2$ is selected from
gly-ser-gly-leu and
gly-ala-gly-leu.

54. Use according to Claim 49 wherein $X^2$ is a fragment consisting of two amino-acid residues.

55. Use according to Claim 54 wherein $X^2$ is gly-leu.

56. Use according to Claim 43 wherein n is one.

57. Use according to Claim 43 wherein $X^3$ is a peptidic fragment consisting of one to six amino-acid residues and p is zero or one.

58. Use according to Claim 57 wherein $X^3$ is a fragment consisting of four or five amino-acid residues.

59. Use according to Claim 58 wherein $X^3$ is asn-ser-phe-arg-tyr.

60. Use according to Claim 58 wherein $X^3$ is a fragment consisting of four amino-acid residues selected from asn, ser, phe and arg.

61. Use according to Claim 60 wherein $X^3$ is asn-ser-phe-arg.

62. Use according to Claim 43 wherein r is zero.

63. Use according to Claim 43 wherein
$X^1$ is selected from
arg-arg,
arg-ser and
ser-ser;
$X^2$ is selected from
gly-ser-gly-leu,
gly-ala-gly-leu,
gln-ser-gly-leu,
gly-ala-gln-ser,
gly-ala-gln-leu,
gly-ser,
gln-leu,
gly-ala and
gly-leu;
$X^3$ is selected from
asn-ser-phe,
asn-ser-phe-arg,
lys-ser-phe-arg,
asn-ser-phe-arg-tyr,
lys-ser-phe-arg-tyr,
asn-ser-phe-arg-$^{125}$I-tyr and
lys-ser-phe-arg-$^{125}$I-tyr. and wherein m is zero or one, n is one and p is zero or one.

64. Use according to Claim 63 wherein $X^1$ is selected from
arg-arg,
arg-ser and
ser-ser;

$X^2$ is selected from
gly-ser-gly-leu,
gly-ser,
gln-leu and
gly-ala;
$X^3$ is selected from
asn-ser-phe-arg,
lys-ser-phe-arg,
asn-ser-phe-arg-tyr,
lys-ser-phe-arg-tyr,
asn-ser-phe-arg-$^{125}$I-tyr and
lys-ser-phe-arg-$^{125}$I-tyr

65. Use according to Claim 64 wherein
$X^1$ is selected from
arg-arg,
arg-ser and
ser-ser;
$X^2$ is gly-ser-gly-leu;
$X^3$ is selected from
asn-ser-phe-arg-tyr,
lys-ser-phe-arg-tyr,
asn-ser-phe-art-$^{125}$I-tyr and
lys-ser-phe-arg-$^{125}$I-tyr.

66. Use according to Claim 65 wherein said peptide is:

**cys-gly-arg-ile-asp-arg-ile-gly-ser-gly-leu-gly-cys-$NH_2$.**

67. Use according to Claim 65 wherein said peptide is:

**ser-ser-cys-gly-arg-ile-asp-arg-ile-gly-ser-gly-leu-gly-cys-asn-**

**ser-phe-arg-tyr.**

68. Use according to Claim 64 wherein
$X^1$ is selected from
arg-arg,
arg-ser and
ser-ser;
$X^2$ is selected from
gly-ser,
gly-leu,
gln-leu and
gly-ala;
$X^3$ is selected from
asn-ser-phe-arg,
lys-ser-phe-arg,
asn-ser-phe-arg-tyr,
lys-ser-phe-arg-tyr,
asn-ser-phe-arg-$^{125}$I-tyr and
lys-ser-phe-arg-$^{125}$I-tyr.

69. Use according to Claim 68 wherein said B terminus is a primary amide moiety or a lower alkyl secondary or tertiary amide moiety when $X^3$ is selected from
asn-ser-phe-arg and
lys-ser-phe-arg.

70. Use according to Claim 64 wherein
$X^1$ is selected from
arg-arg,
arg-ser and
ser-ser;
$X^2$ is gly-ser-gly-leu;
$X^3$ is selected from
asn-ser-phe-arg and
lys-ser-phe-arg.

71. Use according to Claim 70 wherein said peptide is

**cys-gly-arg-ile-asp-arg-ile-gly-ser-gly-leu-gly-cys-asn-ser-phe-**
**arg-$NH_2$.**

72. Use according to Claim 63 wherein
$X^1$ is selected from
arg-arg,
arg-ser and
ser-ser;
$X^2$ is selected from
gly-ser-gly-leu,
gly-ala-gly-leu,
gln-ser-gly-leu,
gly-ala-gln-ser,
gly-ala-gln-leu,
gly-leu,
gly-ala,
gly-ser and
gln-leu;
$X^3$ is selected from
asn-ser-phe-arg and
lys-ser-phe-arg;
and wherein each of m, n and p is one.

73. Use according to Claim 72 wherein
$X^1$ is ser-ser;
$X^2$ is selected from
gly-ala-gly-leu,
gly-ser-gly-leu,
gln-ser-gly-leu,
gly-ala-gln-ser and
gly-ala-gln-leu;
$X^3$ is asn-ser-phe-arg.

74. Use according to Claim 73 wherein said peptide is

**ser-ser-cys-gly-arg-ile-asp-arg-ile-gln-ser-gly-leu-gly-cys-asn-ser-**
**phe-arg-$NH_2$.**

75. Use according to Claim 73 wherein said peptide is

**ser-ser-cys-gly-arg-ile-asp-arg-ile-gly-ala-gln-ser-gly-cys-asn-ser-**
**phe-arg-$NH_2$.**

76. Use according to Claim 73 wherein said peptide is

**ser-ser-cys-gly-arg-ile-asp-arg-ile-gly-ala-gln-leu-gly-cys-asn-ser-phe-arg-$NH_2$.**

77. Use according to Claim 73 wherein said peptide is

**ser-ser-cys-gly-arg-ile-asp-arg-ile-gly-ser-gly-leu-gly-cys-asn-ser-phe-arg-$NH_2$.**

78. Use according to Claim 73 wherein peptide is

**ser-ser-cys-gly-arg-ile-asp-arg-ile-gly-ala-gly-leu-gly-cys-asn-ser-phe-arg-$NH_2$.**

79. Use according to Claim 72 wherein
$X^1$ is selected from
arg-arg,
arg-ser and
ser-ser;
$X^2$ is selected from
gln-leu,
gly-ser,
gly-ala and
gly-leu;
$X^3$ is selected from
asn-ser-phe-arg and
lys-ser-phe-arg;
and wherein each of m, n and p is one.

80. Use according to Claim 79 wherein
$X^1$ is ser-ser;
$X^2$ is selected from
gln-leu,
gly-ser,
gln-ala and
gly-leu;
$X^3$ is selected from
asn-ser-phe-arg and
lys-ser-phe-arg.

81. Use according to Claim 80 wherein said peptide is

**ser-ser-cys-gly-arg-ile-asp-arg-ile-gln-leu-gly-cys-asn-ser-phe-arg-$NH_2$.**

82. Use according to Claim 80 wherein said peptide is

**ser-ser-cys-gly-arg-ile-asp-arg-ile-gly-ser-gly-cys-asn-ser-phe-arg-$NH_2$.**

83. Use according to Claim 80 wherein said peptide is

ser-ser-cys-gly-arg-ile-asp-arg-ile-gly-ala-gly-cys-asn-ser-phe-arg-$NH_2$.

84. Use according to Claim 80 wherein said peptide is

ser-ser-cys-gly-arg-ile-asp-arg-ile-gly-leu-gly-cys-asn-ser-phe-arg-$NH_2$.

85. A peptide of the formula

$$A-X^1_m-cys-gly-arg-ile-asp-arg-ile-X^2_n-gly-cys-X^3_r-B \quad (II)$$

(P') (P')

wherein

$X^1$ is a peptidic fragment consisting of one to about eight amino-acid residues selected from arg, ser, pro and leu, and wherein m is an integer selected from zero through four;

$X^2$ is a peptidic fragment consisting of one to about six amino-acid residues selected from gly, leu, ser, ala and gln, and wherein n is an integer selected from zero through three;

$X^3$ is a peptidic fragment consisting of one to about six amino-acid residues selected from lys, asn, ser, phe, arg and tyr, wherein r is an integer selected from zero through four;

wherein A represents an amino terminus or its pharmaceutically-acceptable salt and wherein B represents a carboxyl terminus or its pharmaceutically-acceptable ester, amide or salt; wherein (P') is a protecting group used as protection for the sulfhydryl function of the cysteine residue; and wherein the cysteine residues may or may not be simultaneously protected; with the proviso that the sum of all amino-acid residues in said peptide is an integer in a range from about 13 through about 25.

86. Peptide of Claim 85 wherein $X^1$ is selected from arg-arg, arg-ser and ser-ser; wherein $X^2$ is selected from gly-ser-gly-leu, gly-ala-gly-leu, gln-ser-gly-leu, gly-ala-gln-ser, gly-ala-gln-leu, gly-ser, gly-leu, gln-leu and gly-ala; wherein $X^3$ is selected from asn-ser-phe, lys-ser-phe, asn-ser-phe-arg, lys-ser-phe-arg, asn-ser-phe-arg-tyr and lys-ser-phe-arg-tyr; wherein m is zero or one, n is one, and r is zero or one; and wherein (P') is an acetamidomethyl group.

Claims for the following Contracting State: ES

1. Process for the preparation of a peptide of the formula

$$A-X^1_m-cys-gly-arg-ile-asp-arg-ile-X^2_n-gly-cys-X^3_p-B$$

wherein

$X^1$ is a fragment consisting of one to about eight amino-acid residues selected from arg, ser, pro and leu, and wherein m is an integer selected from zero through four;

$X^2$ is a fragment consisting of one to about six amino-acid residues selected from gly, leu, ser, ala and gln, and wherein n is an integer selected from zero through three;

$X^3$ is a fragment consisting of one to about six amino-acid residues selected from lys, asn, ser, phe, arg, tyr and $^{125}$I-tyr, wherein p is an integer selected from zero through four;

wherein A represents an amino terminus or its pharmaceutically-acceptable salt and wherein B represents a carboxyl terminus or its pharmaceutically-acceptable ester, amide or salt;

with the proviso that the sum of all amino-acid residues in said peptides is an integer in a range from about 13 through about 25,

characterized in that

A) an appropriate N-protected α-amino acid is coupled to a suitable resin to give

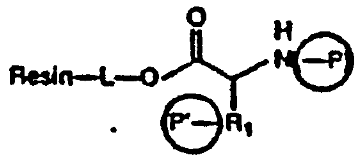

wherein (P) is an appropriate protecting group of the amino acid function and (P') is a protecting group of a function on a side chain, both known to be useful in the art in the step-wise synthesis of polypeptides, and wherein $R_1$ being the side chain group of the desired α-amino acid as defined before;
said resin coupled starting α-amino acid then being deprotected, washed and subsequently coupled in a known manner to appropriate activated α-amino acids of the general formula

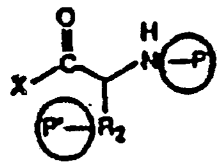

wherein (P') , (P) are suitable protecting groups, $R_2$ being the side chain group of the α-amino acid and X is an appropriate activating group, to give the desired amino acid sequence coupled to the resin having the general formula

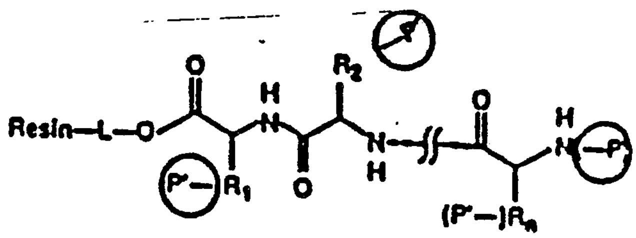

with (P), (P') as defined and $R_1$, $R_2$, $R_n$ being the side chain groups of the desired α-amino acids, said peptide subsequently being removed from the resin and deprotected in a known manner to give the intermediate linear peptides of the general formula

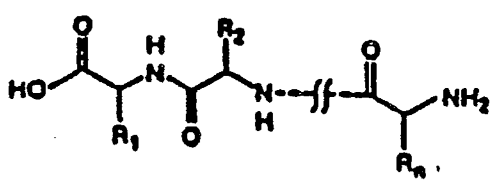

with $R_1$, $R_2$, $R_n$ being the side chain groups of the coupled α-amino acids as defined before;
said linear peptide subsequently being oxidized in a known manner to yield the desired cyclic compounds,

or B) that the desired peptides are prepared in a manner known per se by partial solid phase techniques; by fragment condensation or by classical solution addition using the appropriate α-amino acids;

or C) that the desired peptides are prepared in a known manner by transforming a microorganism using an expression vector including a promotor and operator together with such a structural gene and causing such a transformed microorganism to express the peptide.

2. Process according to Claim 1 wherein $X^1$ of the peptide prepared is a fragment consisting of one to eight amino-acid residues and m is zero or one.

3. Process according to Claim 2 wherein $X^1$ is a peptidic fragment consisting of four or six amino-acid residues selected from ser, arg and leu.

4. Process according to Claim 3 wherein $X^1$ is a fragment consisting of two to four amino-acid residues selected from ser and arg.

5. Process according to Claim 4 wherein $X^1$ is a fragment consisting of one or two serine residues.

6. Process according to Claim 1 wherein m is zero.

7. Process according to Claim 1 wherein $X^2$ is a fragment consisting of two to six amino-acid residues and n is one.

8. Process. according to Claim 7 wherein $X^2$ is a peptidic fragment consisting of two or four amino-acid residues.

9. Process according to Claim 8 wherein $X^2$ is selected from
gly-ala-gly-leu,
gly-ala-gln-leu,
gln-ser-gly-leu,
gly-ala-gln-ser,
gln-leu,
gly-ser and
gly-ala.

10. Process according to Claim 8 wherein $X^2$ is a fragment consisting of four amino-acid residues.

11. Process according to Claim 10 wherein $X^2$ is selected from
gly-ser-gly-leu and
gly-ala-gly-leu.

12. Process according to Claim 8 wherein $X^2$ is a fragment consisting of two amino-acid residues.

13. Process according to Claim 12 wherein $X^2$ is gly-leu.

14. Process according to Claim 1 wherein n is one.

15. Process according to Claim 1 wherein $X^3$ is a peptidic fragment consisting of one to six amino-acid residues and p is zero or one.

16. Process according to Claim 15 wherein $X^3$ is a fragnent consisting of four or five amino-acid residues.

17. Process according to Claim 16 wherein $X^3$ is asn-ser-phe-arg-tyr.

18. Process according to Claim 16 wherein $X^3$ is a fragment consisting of four amino-acid residues selected from asn, ser, phe and arg.

19. Process according to Claim 18 wherein $X^3$ is asn-ser-phe-arg.

20. Process according to Claim 1 wherein r is zero.

21. Process according to Claim 1 wherein $X^1$ is selected from
arg-arg,
arg-ser and
ser-ser;
$X^2$ is selected from
gly-ser-gly-leu,
gly-ala-gly-leu,
gln-ser-gly-leu,
gly-ala-gln-ser,
gly-ala-gln-leu,
gly-ser,
gln-leu,
gly-ala and
gly-leu;
$X^3$ is selected from
asn-ser-phe,
asn-ser-phe-arg,
lys-ser-phe-arg,
asn-ser-phe-arg-tyr,
lys-ser-phe-arg-tyr,
asn-ser-phe-arg-$^{125}$I -tyr and
lys-ser-phe-arg-$^{125}$I-tyr.
and wherein m is zero or one, n is one and p is zero or one.

22. Process according to Claim 21 wherein $X^1$ is selected from
arg-arg,
arg-ser and

ser-ser;
$X^2$ is selected from
gly-ser-gly-leu,
gly-ser,
gln-leu and
gly-ala;
$X^3$ is selected from
asn-ser-phe-arg,
lys-ser-phe-arg,
asn-ser-phe-arg-tyr,
lys-ser-phe-arg-tyr,
asn-ser-phe-arg-$^{125}$I-tyr and
lys-ser-phe-arg-$^{125}$I-tyr.

23. Process according to Claim 22 wherein
$X^1$ is selected from
arg-arg,
arg-ser and
ser-ser;
$X^2$ is gly-ser-gly-leu;
$X^3$ is selected from
asn-ser-phe-arg-tyr,
lys-ser-phe-arg-tyr,
asn-ser-phe-arg-$^{125}$I-tyr and
lys-ser-phe-arg-$^{125}$I-tyr.

24. Process according to Claim 23 wherein the peptide prepared is

**cys-gly-arg-ile-asp-arg-ile-gly-ser-gly-leu-gly-cys-$NH_2$.**

25. Process according to Claim 23 wherein the peptide prepared is

**ser-ser-cys-gly-arg-ile-asp-arg-ile-gly-ser-gly-leu-gly-cys-asn-**
**ser-phe-arg-tyr.**

26. Process according to Claim 22 wherein
$X^1$ is selected from
arg-arg,
arg-ser and
ser-ser;
$X^2$ is selected from
gly-ser,
gly-leu,
gln-leu and
gly-ala;
$X^3$ is selected from
asn-ser-phe-arg,
lys-ser-phe-arg,
asn-ser-phe-arg-tyr,
lys-ser-phe-arg-tyr,
asn-ser-phe-arg-$^{125}$I-tyr and
lys-ser-phe-arg-$^{125}$I-tyr.

27. Process according to Claim 26 wherein said B terminus is a primary amide moiety r a lower alkyl secondary or tertiary amide moiety when $X^3$ is selected from
asn-ser-phe-arg and
lys-ser-phe-arg.

28. Process according to Claim 22 wherein
$X^1$ is selected from
arg-arg,
arg-ser and
ser-ser;
$X^2$ is gly-ser-gly-leu;
$X^3$ is selected from
asn-ser-phe-arg and
lys-ser-phe-arg.

29. Process according to Claim 28 wherein the peptide prepared is

**cys-gly-arg-ile-asp-arg-ile-gly-ser-gly-leu-gly-cys-asn-ser-phe-**
**arg-$NH_2$.**

30. Process according to Claim 21 wherein
$X^1$ is selected from
arg-arg,
arg-ser and
ser-ser;
$X^2$ is selected from
gly-ser-gly-leu,
gly-ala-gly-leu,
gln-ser-gly-leu,
gly-ala-gln-ser,
gly-ala-gln-leu,
gly-leu,
gly-ala,
gly-ser and
gln-leu;
$X^3$ is selected from
asn-ser-phe-arg and
lys-ser-phe-arg;
and wherein each of m, n and p is one.

31. Process according to Claim 30 wherein
$X^1$ is ser-ser;
$X^2$ is selected from
gly-ala-gly-leu,
gly-ser-gly-leu,
gln-ser-gly-leu,
gly-ala-gln-ser and
gly-ala-gln-leu;
$X^3$ is asn-ser-phe-arg.

32. Process according to Claim 31 wherein the peptide prepared is

**ser-ser-cys-gly-arg-ile-asp-arg-ile-gln-ser-gly-leu-gly-cys-asn-ser-**
**phe-arg-$NH_2$.**

33. Process according to Claim 31 wherein the peptide prepared is

ser-ser-cys-gly-arg-ile-asp-arg-ile-gly-ala-gln-ser-gly-cys-asn-ser-
phe-arg-$NH_2$.

34. Process according to Claim 31 wherein the peptide prepared is

ser-ser-cys-gly-arg-ile-asp-arg-ile-gly-ala-gln-leu-gly-cys-asn-ser-
phe-arg-$NH_2$.

35. Process according to Claim 31 wherein the peptide prepared is

ser-ser-cys-gly-arg-ile-asp-arg-ile-gly-ser-gly-leu-gly-cys-asn-ser-
phe-arg-$NH_2$.

36. Process according to Claim 31 wherein the peptide prepared is

ser-ser-cys-gly-arg-ile-asp-arg-ile-gly-ala-gly-leu-gly-cys-asn-ser-
phe-arg-$NH_2$.

37. Process according to Claim 30 wherein
$X^1$ is selected from
arg-arg,
arg-ser and
ser-ser;
$X^2$ is selected from
gln-leu,
gly-ser,
gly-ala and
gly-leu;
$X^3$ is selected from
asn-ser-phe-arg and
lys-ser-phe-arg;
and wherein each of m, n and p is one.

38. Process according to Claim 37 wherein
$X^1$ is ser-ser;
$X^2$ is selected from
gln-leu,
gly-ser,
gln-ala and
gly-leu;
$X^3$ is selected from
asn-ser-phe-arg and
lys-ser-phe-arg.

39. Process according to Claim 38 wherein the peptide prepared is

ser-ser-cys-gly-arg-ile-asp-arg-ile-gln-leu-gly-cys-asn-ser-phe-arg-$NH_2$.

40. Process according to Claim 38 wherein the peptide prepared is

**ser-ser-cys-gly-arg-ile-asp-arg-ile-gly-ser-gly-cys-asn-ser-phe-arg-$NH_2$.**

41. Process according to Claim 38 wherein the peptide prepared is

**ser-ser-cys-gly-arg-ile-asp-arg-ile-gly-ala-gly-cys-asn-ser-phe-arg-$NH_2$.**

42. Process according to Claim 38 wherein the peptide prepared is

**ser-ser-cys-gly-arg-ile-asp-arg-ile-gly-leu-gly-cys-asn-ser-phe-arg-$NH_2$.**

43. Use of a therapeutically-effective amount of a peptide of the formula

$$A\text{-}X^1_m\text{-cys-gly-arg-ile-asp-arg-ile-}X^2_n\text{-gly-cys-}X^3_p\text{-}B$$

wherein
$X^1$ is a fragment consisting of one to about eight amino-acid residues selected from arg, ser, pro and leu, and Wherein m is an integer selected from zero through four;
$X^2$ is a fragment consisting of one to about six amino-acid residues selected from gly, leu, ser, ala and gln, and wherein n is an integer selected from zero through three;
$X^3$ is a fragment consisting of one to about six amino-acid residues selected from lys, asn, ser, phe, arg, tyr and $^{125}$I-tyr, wherein p is an integer selected from zero through four;
wherein A represents an amino terminus or its pharmaceutically-acceptable salt and wherein B represents a carboxyl terminus or its pharmaceutically-acceptable ester, amide or salt;
with the proviso that the sum of all amino-acid residues in said peptide is an integer in a range from about 13 through about 25, for the manufacture of a medicament for treating hypertension.

44. Use according to Claim 43 wherein $X^1$ of the peptide is a fragment consisting of one to eight amino-acid residues and m is zero or one.

45. Use according to Claim 44 wherein $X^1$ is a peptidic fragment consisting of four or six amino-acid residues selected from ser, arg and leu.

46. Use according to Claim 45 wherein $X^1$ is a fragment consisting of two to four amino-acid residues selected from ser and arg.

47. Use according to Claim 46 wherein $X^1$ is a fragment consisting of one or two serine residues.

48. Use according to Claim 43 wherein m is zero.

49. Use according to Claim 43 wherein $X^2$ is a fragment consisting of two to six amino-acid residues and n is one.

50. Use according to Claim 49 wherein $X^2$ is a peptidic fragment consisting of two or four amino-acid residues.

51. Use according to Claim 50 wherein $X^2$ is selected from
gly-ala-gly-leu,
gly-ala-gln-leu,
gln-ser-gly-leu,
gly-ala-gln-ser,
gln-leu,
gly-ser and
gly-ala.

52. Use according to Claim 49 wherein $X^2$ is a fragment consisting of four amino-acid residues.

53. Use according to Claim 52 wherein $X^2$ is selected from
gly-ser-gly-leu and
gly-ala-gly-leu.

54. Use according to Claim 49 wherein $X^2$ is a fragment consisting of two amino-acid residues.

55. Use according to Claim 54 wherein $X^2$ is gly-leu.

56. Use according to Claim 43 wherein n is one.

57. Use according to Claim 43 wherein $X^3$ is a peptidic fragment consisting of one to six amino-acid residues and p is zero or one.

58. Use according to Claim 57 wherein $X^3$ is a fragment consisting of four or five amino-acid residues.

59. Use according to Claim 58 wherein $X^3$ is asn-ser-phe-arg-tyr.

60. Use according to Claim 58 wherein $X^3$ is a fragment consisting of four amino-acid residues selected from asn, ser, phe and arg.

61. Use according to Claim 60 wherein $X^3$ is asn-ser-phe-arg.

62. Use according to Claim 43 wherein r is zero.

63. Use according to Claim 43 wherein
$X^1$ is selected from
arg-arg,
arg-ser and
ser-ser;
$X^2$ is selected from
gly-ser-gly-leu,
gly-ala-gly-leu,
gln-ser-gly-leu,
gly-ala-gln-ser,
gly-ala-gln-leu,
gly-ser,
gln-leu,
gly-ala and
gly-leu;
$X^3$ is selected from
asn-ser-phe,
asn-ser-phe-arg,
lys-ser-phe-arg,
asn-ser-phe-arg-tyr,
lys-ser-phe-arg-tyr,
asn-ser-phe-arg-$^{2125}$I-tyr and
lys-ser-phe-arg-$^{125}$I-tyr.
and wherein m is zero or one, n is one and p is zero or one.

64. Use according to Claim 63 wherein
$X^1$ is selected from
arg-arg,
arg-ser and
ser-ser;
$X^2$ is selected from
gly-ser-gly-leu,
gly-ser,
gln-leu and
gly-ala;
$X^3$ is selected from
asn-ser-phe-arg,
lys-ser-phe-arg,
asn-ser-phe-arg-tyr,
lys-ser-phe-arg-tyr,
asn-ser-ph-arg-$^{125}$I-tyr and
lys-ser-phe-arg-$^{125}$I-tyr.

65. Use according to Claim 64 wherein
X is selected from
arg-arg,

arg-ser and
ser-ser;
$X^2$ is gly-ser-gly-leu;
$X^3$ is selected from
asn-ser-phe-arg-tyr,
lys-ser-phe-arg-tyr
asn-ser-phe-arg-$^{125}$I-tyr and
lys-ser-phe-arg-$^{125}$I-tyr.

66. Use according to Claim 65 wherein said peptide is:

**cys-gly-arg-ile-asp-arg-ile-gly-ser-gly-leu-gly-cys-$NH_2$.**
|________________________________________|

67. Use according to Claim 65 wherein said peptide is:

**ser-ser-cys-gly-arg-ile-asp-arg-ile-gly-ser-gly-leu-gly-cys-asn-**
|________________________________________|
**ser-phe-arg-tyr.**

68. Use according to Claim 64 wherein
$X^1$ is selected from
arg-arg,
arg-ser and
ser-ser;
$X^2$ is selected from
gly-ser,
gly-leu,
gln-leu and
gly-ala;
$X^3$ is selected from
asn-ser-phe-arg,
lys-ser-phe-arg,
asn-ser-phe-arg-tyr
, lys-ser-phe-arg-tyr,
asn-ser-phe-arg-$^{125}$I-tyr and
lys-ser-phe-arg-$^{125}$I-tyr.

69. Use according to Claim 68 wherein said B terminus is a primary amide moiety or a lower alkyl secondary or tertiary amide moiety when $X^3$ is selected from
asn-ser-phe-arg and
lys-ser-phe-arg.

70. Use according to Claim 64 wherein
$X^1$ is selected from
arg-arg,
arg-ser and
ser-ser;
$X^2$ is gly-ser-gly-leu;
$X^3$ is selected from
asn-ser-phe-arg and
lys-ser-phe-arg.

71. Use according to Claim 70 wherein said peptide is

**cys-gly-arg-ile-asp-arg-ile-gly-ser-gly-leu-gly-cys-asn-ser-phe-**
**|________________________________________________|**
**arg-$NH_2$.**

72. Use according to Claim 63 wherein
X¹ is selected from
arg-arg,
arg-ser and
ser-ser;
X² is selected from
gly-ser-gly-leu,
gly-ala-gly-leu,
gln-ser-gly-leu,
gly ala-gln-ser,
gly-ala-gln-leu,
gly-leu,
gly-ala,
gly-ser and
gln-leu;
X³ is selected from
asn-ser-phe-arg and
lys-ser-phe-arg;
and wherein each of m, n and p is one.

73. Use according to Claim 72 wherein
X¹ is ser-ser;
X² is selected from
gly-ala-gly-leu,
gly-ser-gly-leu,
gln-ser-gly-leu,
gly-ala-gln-ser and
gly-ala-gln-leu;
X³ is asn-ser-phe-arg.

74. Use according to Claim 73 wherein said peptide is

**ser-ser-cys-gly-arg-ile-asp-arg-ile-gln-ser-gly-leu-gly-cys-asn-ser-**
**|________________________________________________|**
**phe-arg-$NH_2$.**

75. Use according to Claim 73 wherein said peptide is

**ser-ser-cys-gly-arg-ile-asp-arg-ile-gly-ala-gln-ser-gly-cys-asn-ser-**
**|________________________________________________|**
**phe-arg-$NH_2$.**

76. Use according to Claim 73 wherein said peptide is

**ser-ser-cys-gly-arg-ile-asp-arg-ile-gly-ala-gln-leu-gly-cys-asn-ser-**
**|________________________________________________|**
**phe-arg-$NH_2$.**

77. Use according to Claim 73 wherein said peptide is

**ser-ser-cys-gly-arg-ile-asp-arg-ile-gly-ser-gly-leu-gly-cys-asn-ser-phe-arg-$NH_2$.**

78. Use according to Claim 73 wherein peptide is

**ser-ser-cys-gly-arg-ile-asp-arg-ile-gly-ala-gly-leu-gly-cys-asn-ser-phe-arg-$NH_2$.**

79. Use according io Claim 72 wherein
X is selected from
arg-arg,
arg-ser and
ser-ser;
$X^2$ is selected from
gln-leu,
gly-ser,
gly-ala and
gly-leu;
$X^3$ is selected from
asn-ser-phe-arg and
lys-ser-phe-arg;
and wherein each of m, n and p is one.

80. Use according to Claim 79 wherein $X^1$ is ser-ser;
$X^2$ is selected from
gln-leu,
gly-ser,
gln-ala and
gly-leu;
$X^3$ is selected from
asn-ser-phe-arg and
lys-ser-phe-arg.

81. Use according to Claim 80 wherein said peptide is

**ser-ser-cys-gly-arg-ile-asp-arg-ile-gln-leu-gly-cys-asn-ser-phe-arg-$NH_2$.**

82. Use according to Claim 80 wherein said peptide is

**ser-ser-cys-gly-arg-ile-asp-arg-ile-gly-ser-gly-cys-asn-ser-phe-arg-$NH_2$.**

83. Use according to Claim 80 wherein said peptide is

**ser-ser-cys-gly-arg-ile-asp-arg-ile-gly-ala-gly-cys-asn-ser-phe-arg-$NH_2$.**

84. Use according to Claim 80 wherein said peptide is

ser-ser-cys-gly-arg-ile-asp-arg-ile-gly-leu-gly-cys-asn-ser-phe-arg-$NH_2$.

85. Process for preparing a peptide of the formula

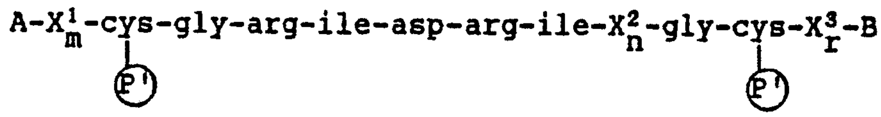 (II)

wherein
X' is a peptidic fragment consisting of one to about eight amino-acid residues selected from arg, ser, pro and leu, and wherein m is an integer selected from zero through four;
$X^2$ is a peptidic fragment consisting of one to about six amino-acid residues selected from gly, leu, ser, ala and gln, and wherein n is an integer selected from zero through three;
$X^3$ is a peptidic fragment consisting of one to about six amino-acid residues selected from lys, asn, ser, phe, arg and tyr, wherein r is an integer selected from zero through four;
wherein A represents an amino terminus or its pharmaceutically-acceptable salt and wherein B represents a carboxyl terminus or its pharmaceutically-acceptable ester, amide or salt; wherein (P') is a protecting group used as protection for the sulfhydryl function of the cysteine residue; and wherein the cysteine residues may or may not be simultaneously protected; with the Proviso that the sum of all amino-acid residues in said peptide is an integer in a range from about 13 through about 25;
characterized in that
an appropriate N-protected α-amino acid is coupled to a suitable resin to give

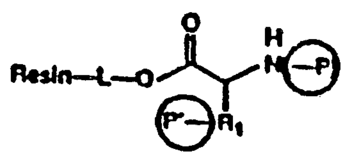

wherein (P) is an appropriate protecting group of the amino acid function and (P') is a protecting group of a function on a side chain, both being known to be useful in the art in the step-wise synthesis of polypeptides, and wherein $R_1$ being the side chain group of the desired amino acid as defined before ;
said resin-coupled starting α-amino acid then being deprotected, washed and subsequently coupled in a known manner with appropriate activated α-amino acids of the general formula

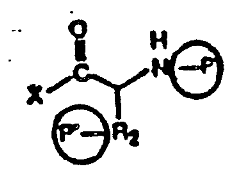

with (P) and (P') as defined above and $R^2$ being the side chain of the α-amino acid and X being an appropriate activating group,
to give the desired amino-acid sequence coupled to the resin and having the general formula

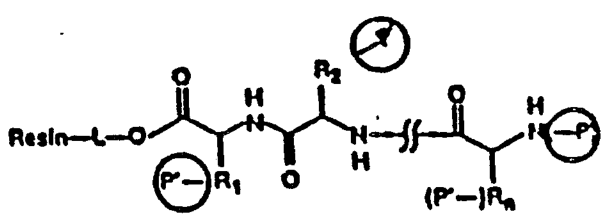

with Ⓟ , (P') as defined and $R_1$, $R_2$, $R_n$ being the side chain groups of the desired α-amino acids as defined before,
said linear peptide subsequently being removed from the resin and optionally being hydrolyzed and transformed on the carboxyl terminus in a known manner to yield the desired compounds.

86. Process according to Claim 85 wherein $X^1$ of the peptide prepared is selected from arg-arg, arg-ser and ser-ser; wherein $X^2$ is selected from gly-ser-gly-leu, gly-ala-gly-leu, gln-ser-gly-leu, gly-ala-gln-ser, gly-ala-gln-leu, gly-ser, gly-leu, gln-leu and gly-ala; wherein $X^3$ is selected from asn-ser-phe, lys-ser-phe, asn-ser-phe-arg, lys-ser-phe-arg, asn-ser-phe-arg-tyr and lys-ser-phe-arg-tyr; wherein m is zero or one, n is one, and r is zero or one; and wherein (P') is an acetamidomethyl group.

GENERAL SCHEME FOR PEPTIDE SOLID PHASE SYNTHESIS

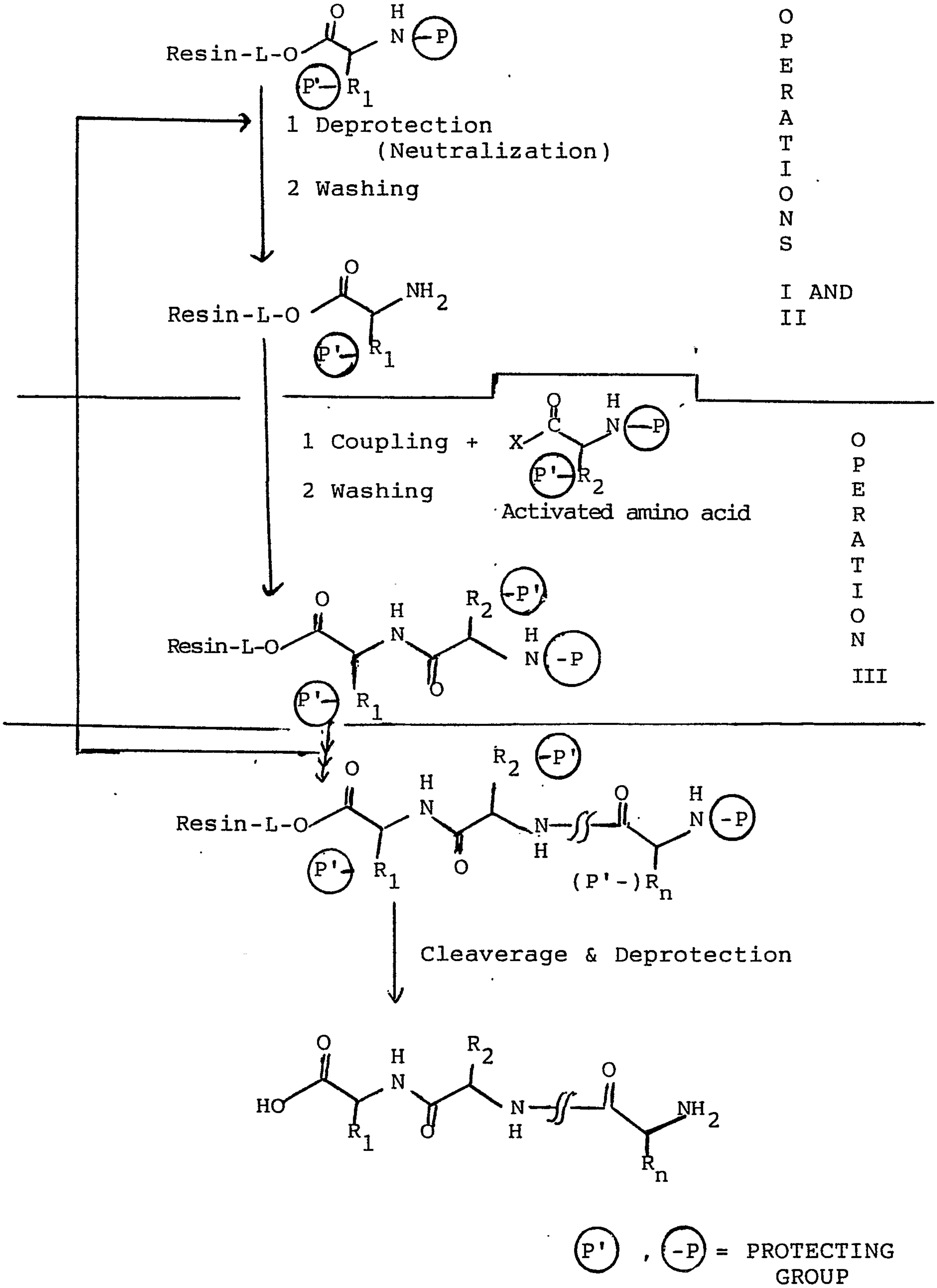


FIG. 1

BINDING DISPLACEMENT CURVES

$^{125}$I-AP-III vs. AP-III and vs. Peptide #9

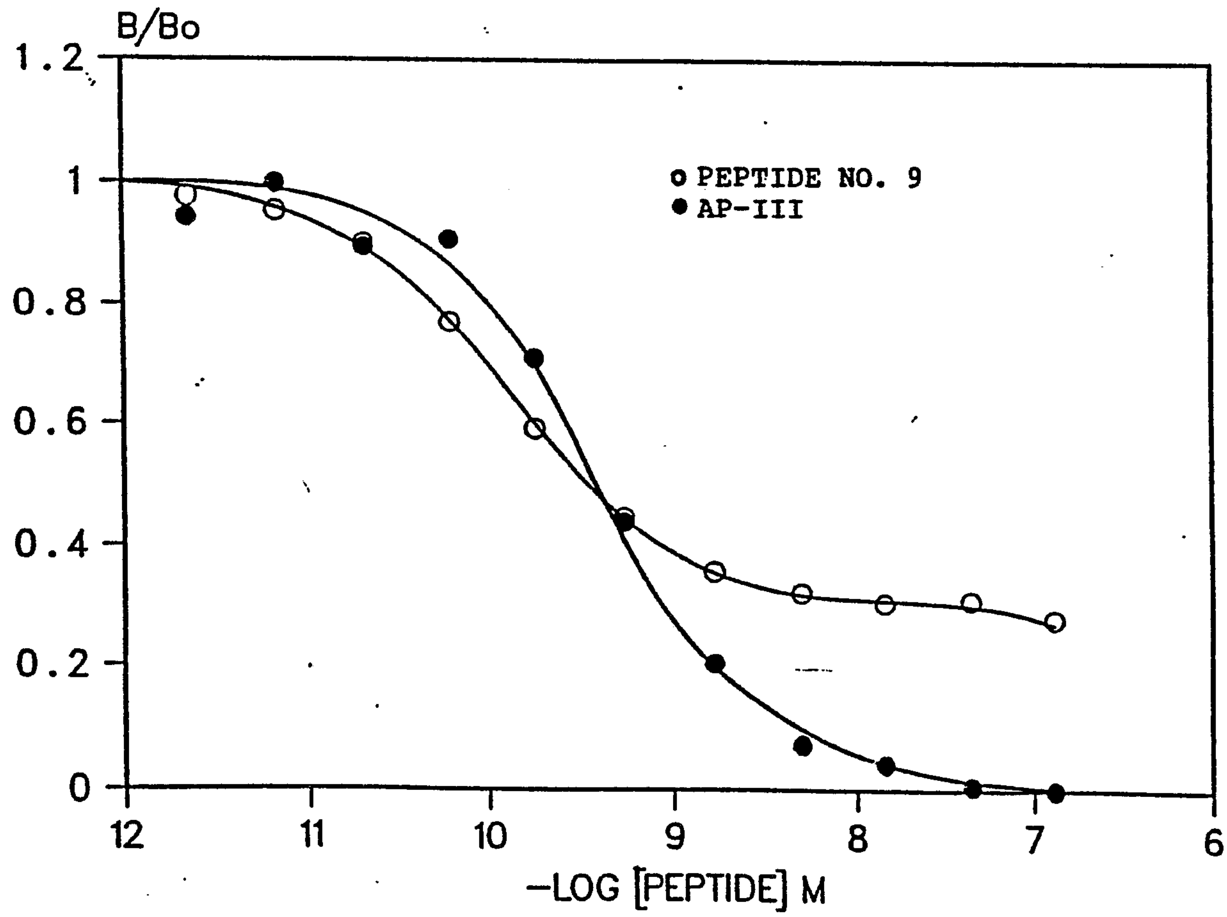


Fig. 2

BINDING DISPLACEMENT CURVES

$^{125}$I-AP-III vs. AP-III and vs. Peptide #10

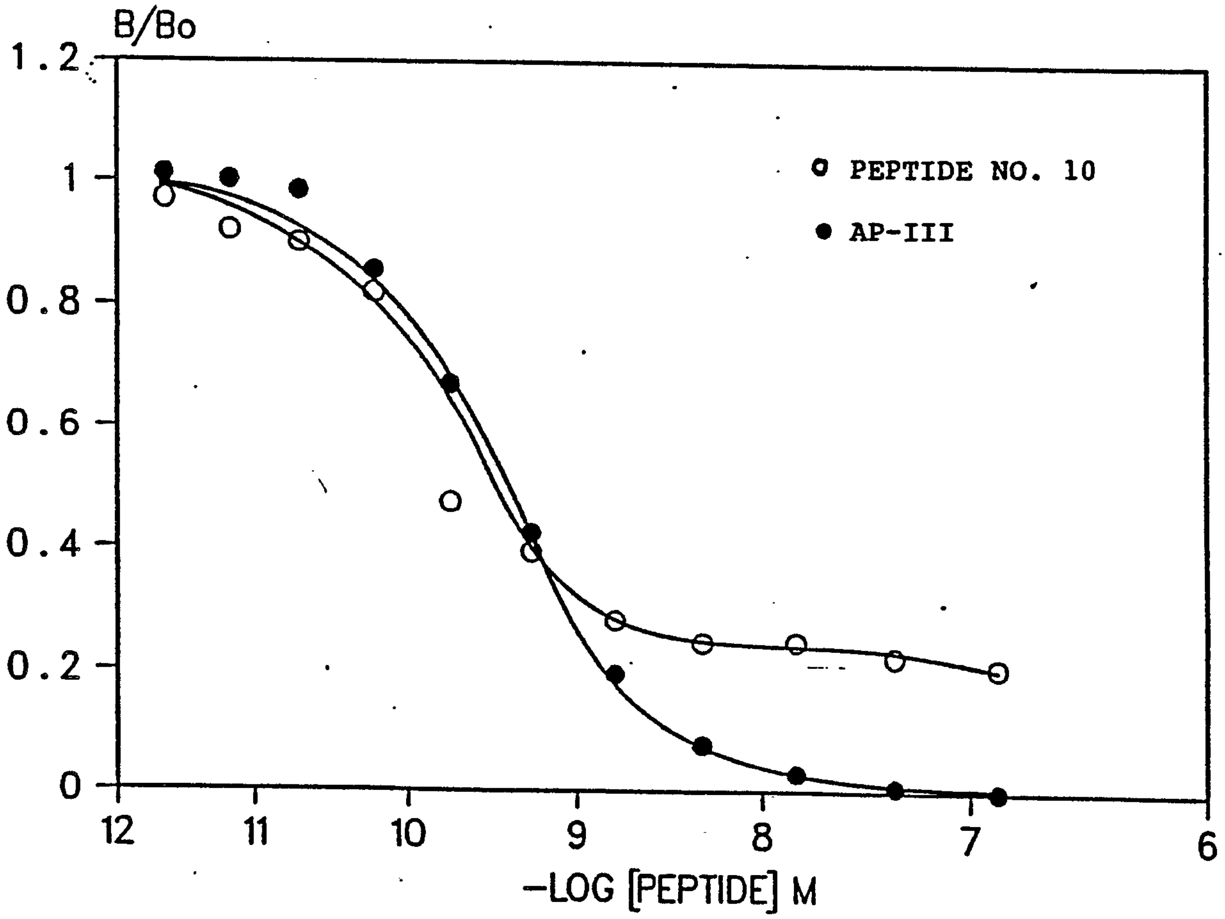


Fig. 3

CURVING DISPLACEMENT CURVES

$^{125}$I-AP-III vs. AP-III and vs. Peptide #12

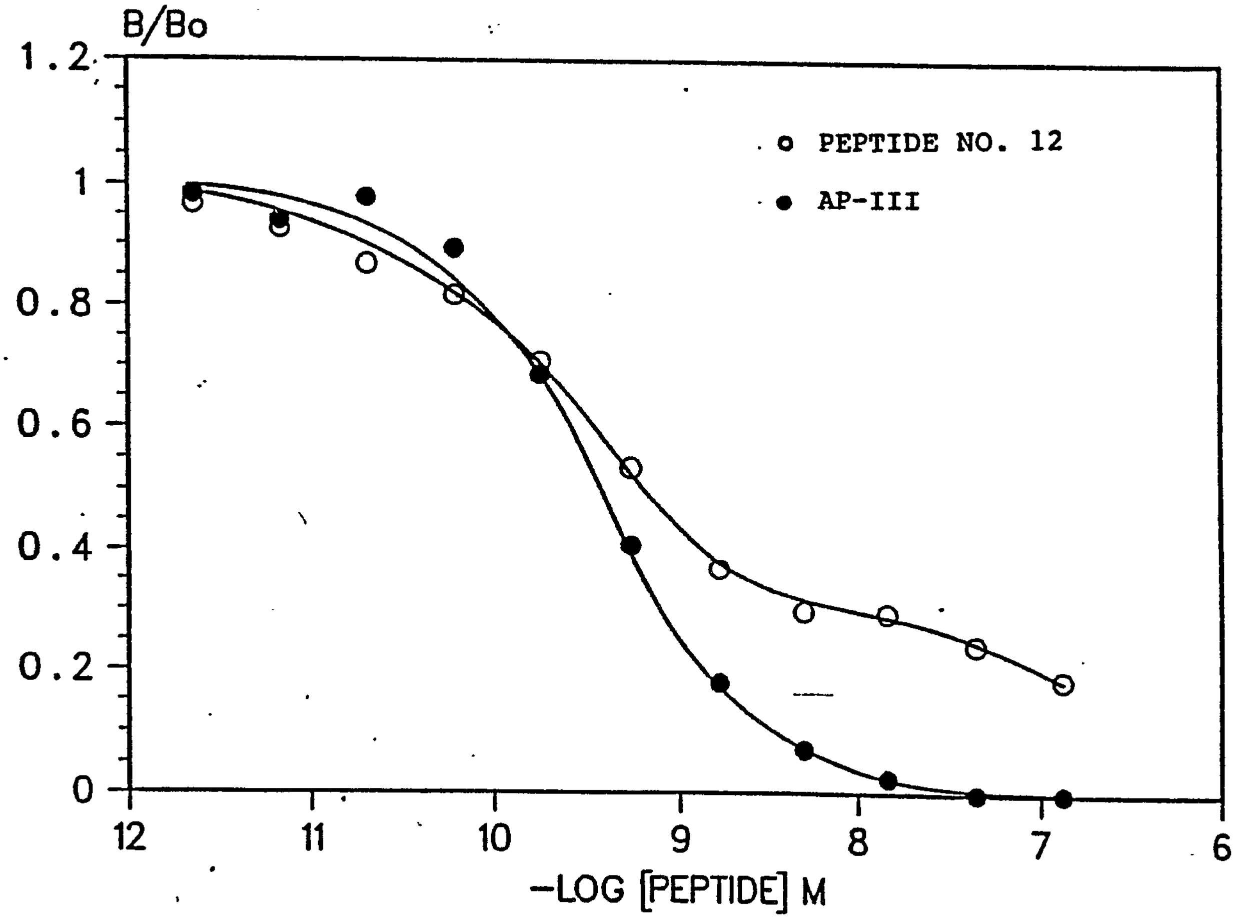


Fig. 4

BINDING DISPLACEMENT CURVES

$^{125}$I-AP-III vs. AP-III and vs. Peptide # 11

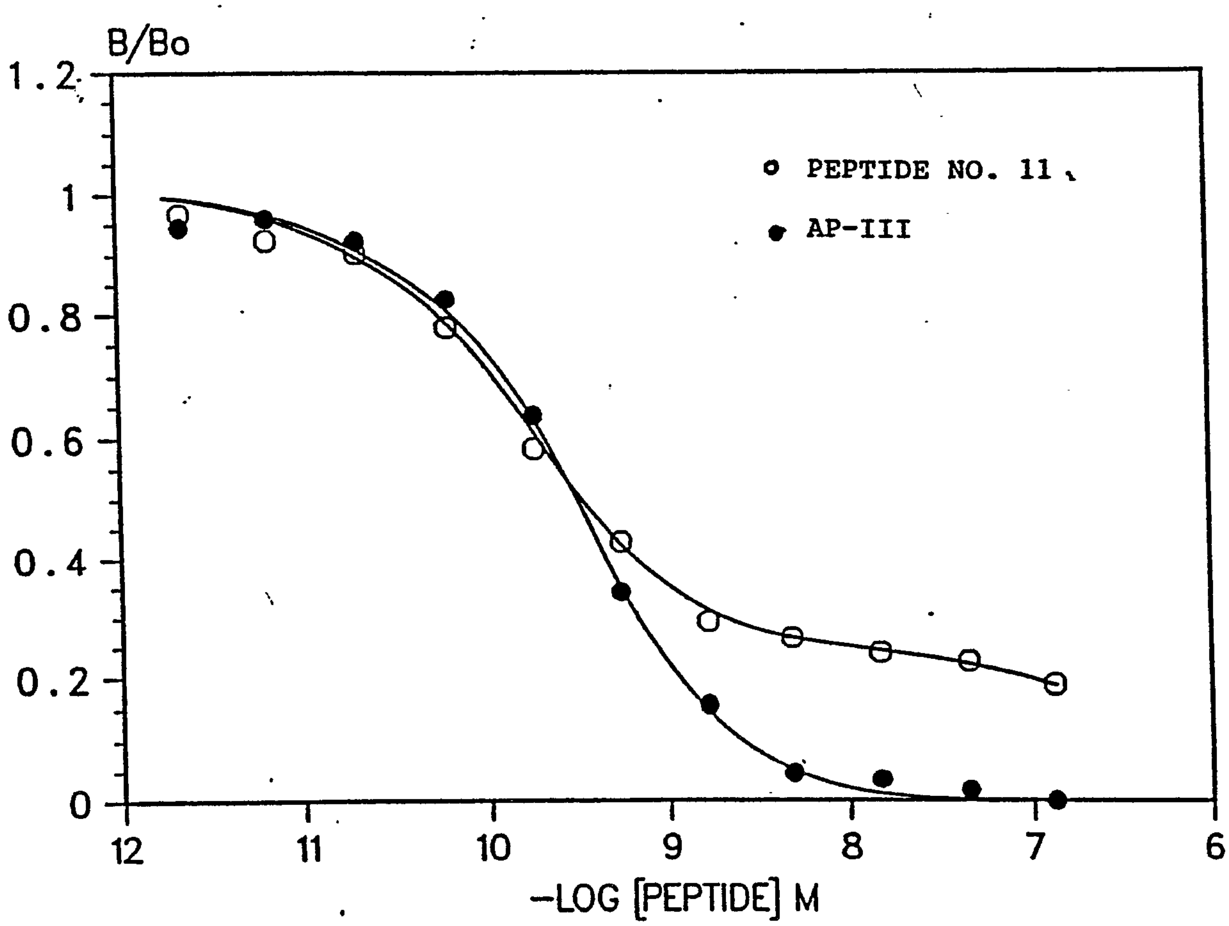


Fig. 5

TIME COURSE OF THE VARIATION OF RENAL EXCRETORY PARAMETERS AND OF MEAN ARTERIAL PRESSURE.

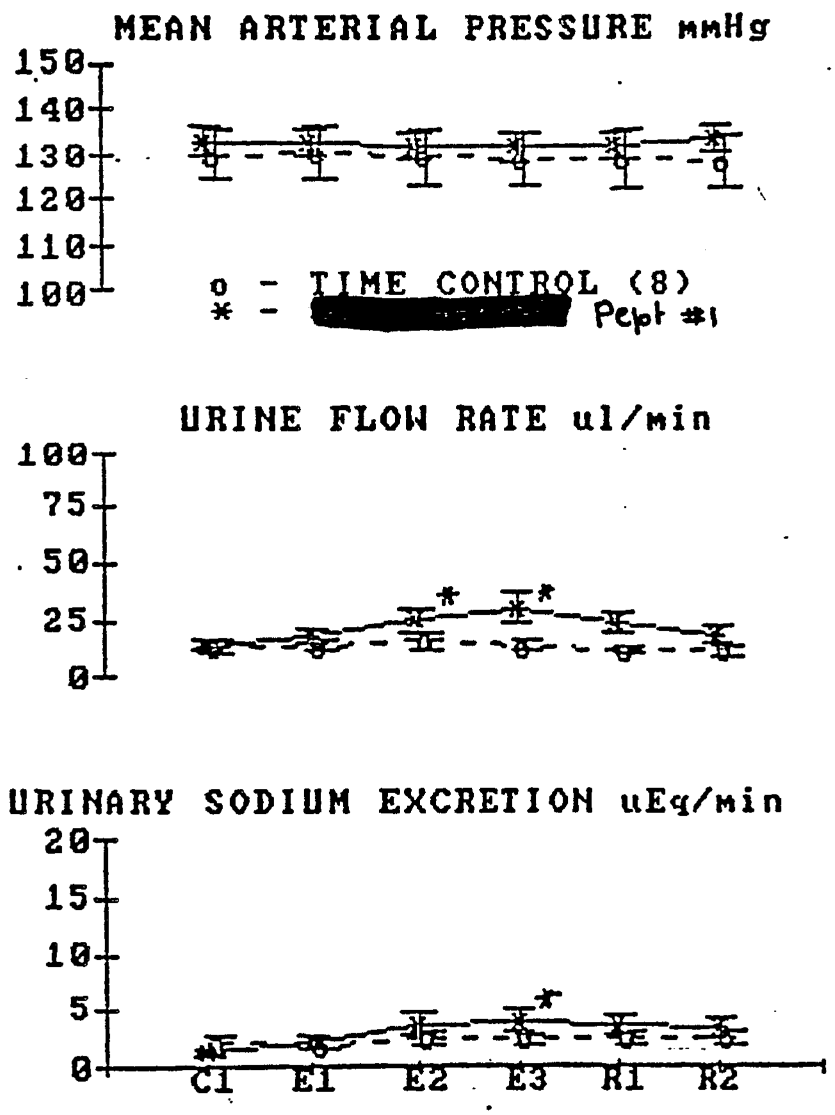


Fig. 6

TIME COURSE OF THE VARIATION OF RENAL EXCRETORY

PARAMETERS AND OF MEAN ARTERIAL PRESSURE.

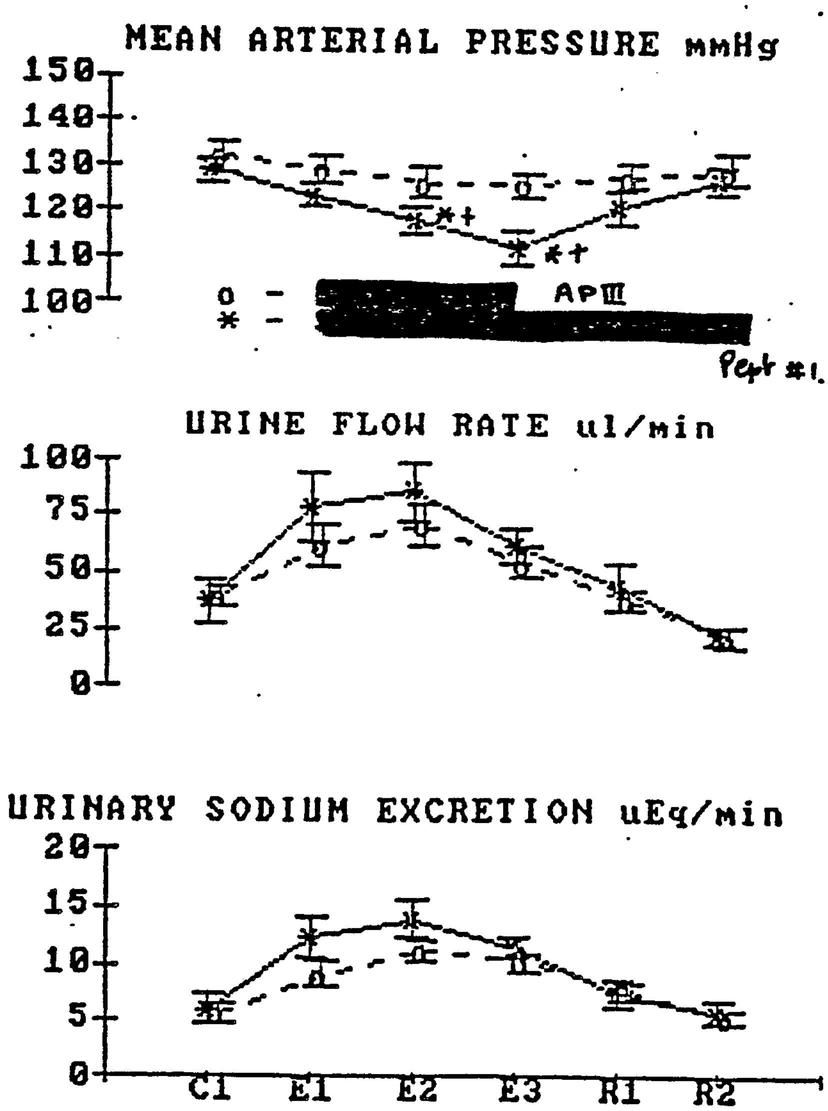


Fig. 7